(19) Europäisches Patentamt
European Patent Office
Office européen des brevets

(11) EP 1 769 746 A1

(12) EUROPEAN PATENT APPLICATION
published in accordance with Art. 158(3) EPC

(43) Date of publication:
04.04.2007 Bulletin 2007/14

(51) Int Cl.:
A61B 8/06 (2006.01)

(21) Application number: 05753303.6

(22) Date of filing: 27.06.2005

(86) International application number:
PCT/JP2005/011763

(87) International publication number:
WO 2006/003866 (12.01.2006 Gazette 2006/02)

(84) Designated Contracting States:
DE FR GB

(30) Priority: 30.06.2004 JP 2004194888

(71) Applicant: Olympus Corporation
Tokyo 151-0072 (JP)

(72) Inventor: MIYAKI, Hironaka
1910065 (JP)

(74) Representative: von Hellfeld, Axel
Wuesthoff & Wuesthoff
Patent- und Rechtsanwälte
Schweigerstrasse 2
81541 München (DE)

(54) ULTRASONIC DIAGNOSIS DEVICE

(57) An ultrasonic diagnostic apparatus transmits ultrasound to an interior of a subject body and receives the ultrasound from the interior of the subject body to obtain plural pieces of ultrasound data, calculates a velocity of a moving body in the subject body as a velocity within a predetermined velocity range based on the plural pieces of ultrasound data, and generates and outputs a velocity image which indicates the calculated velocity as a velocity image of the moving body. The ultrasonic diagnostic apparatus includes an input unit which supplies information indicating a velocity range of interest of the moving body as an input, a velocity range setting control unit, and an image processing control unit. The velocity range setting control unit sets a variable detectable velocity range which is wider than the velocity range of interest and which covers the velocity range of interest as the predetermined velocity range based on the information supplied from the input unit. The image processing control unit allocates color scale data to each velocity within the detectable velocity range, and generates the velocity image based on the allocated color scale data and the calculated velocity.

FIG.1

ULTRASONIC DIAGNOSTIC APPARATUS
1

EP 1 769 746 A1

**Description**

TECHNICAL FIELD

The present invention relates to an ultrasonic diagnostic apparatus which repeatedly performs an ultrasonographic scanning in a direction of every sound ray by irradiating an interior of a living body with ultrasound plural times and sequentially receiving an echo of the ultrasound, and generates and outputs a velocity image, which is a color image indicating a velocity of a moving body in the living body, based on plural pieces of ultrasound data obtained through the ultrasonographic scanning.

BACKGROUND ART

Conventional ultrasonic diagnostic apparatuses perform an ultrasonographic scanning by irradiating an interior of a living body with ultrasound and receiving an echo of the ultrasound to generate and output an ultrasound tomographic image of the interior of the living body and a velocity image which indicates a velocity of a moving body inside the living body. Such conventional ultrasonic diagnostic apparatuses are commonly used as a medical diagnostic apparatus which allows for real-time observation of a tomographic image of a region of interest, such as pathological lesion, inside the living body, or real-time observation of a velocity of the moving body, such as blood. The ultrasonic diagnostic apparatus can find the velocity of the moving body by, for example, carrying out Doppler-method-based processing using ultrasound data obtained through the ultrasonographic scanning of the moving body in the living body. Further, the ultrasonic diagnostic apparatus can generate and output the velocity image, which indicates the velocity of the moving body, using color scale data, in which a certain level of luminance, hue, or the like is allocated to each velocity within a desired velocity range of interest, which is set as an examination target in advance by an operator.

However, when the ultrasonic diagnostic apparatus detects the moving body whose velocity is out of the set velocity range of interest, the ultrasonic diagnostic apparatus ends up displaying the velocity image in an improper level of hue or luminance so as to indicate the velocity and the direction of the moving body in incorrect values (such a phenomenon is called "aliasing"). The aliasing is governed by sampling theorem: aliasing occurs more frequently as the velocity range of interest narrows. When the aliasing occurs, the velocity image displayed by the ultrasonic diagnostic apparatus indicates the velocity and the direction of motion of the moving body at different values from actual values. Therefore, the operator cannot recognize the velocity of the moving body, which is the examination target, correctly; for example, the operator may not be able to recognize a flow rate and a flow direction of a bloodstream correctly. One conventional ultrasonic diagnostic apparatus, which can suppress the occurrence of aliasing, determines whether a velocity range of interest is appropriate for a velocity of a moving body in a region of interest or not, for example, whether a flow rate range is appropriate for a flow rate of a bloodstream of interest or not, based on a number of saturated pixels that become saturated when the flow rate reaches an upper limit of the velocity range of interest, and automatically widens the flow rate range according to a result of determination (see Patent Document 1).

Patent Document 1: Japanese Patent Application Laid-Open No. H11-146879

DISCLOSURE OF INVENTION

PROBLEM TO BE SOLVED BY THE INVENTION

The ultrasonic diagnostic apparatus of the Patent Document 1, however, tends to cause an increase in a cutoff frequency corresponding to the widening of the flow rate range, since the ultrasonic diagnostic apparatus suppresses the occurrence of aliasing by widening the previously set flow rate range, which serves as the velocity range of interest. The cutoff frequency is employed in a filtering process to remove frequency components corresponding to a velocity of a moving body other than the examination target. Hence, the velocity range (velocity range for removal) which is employed in the filtering process to remove the velocity of the moving body other than the examination target is widened along with the widening of the flow rate range, i.e., along with the increase in the cutoff frequency, making a low velocity component within the flow rate range, i.e., an original target of the examination difficult to detect.

In view of the foregoing, an object of the present invention is to provide an ultrasonic diagnostic apparatus which can suppress the occurrence of aliasing without compromising a capability to detect a low velocity component, which is an examination target, within a velocity range of interest.

MEANS FOR SOLVING PROBLEM

In order to solve the problem(s) as described above and to achieve an object, an ultrasonic diagnostic apparatus according to the present invention transmits/receives ultrasound to an interior of a subject body plural times to obtain plural pieces of ultrasound data, generates and outputs an ultrasound tomographic image of the interior of the subject body based on the obtained ultrasound data, calculates a velocity of a moving body that moves in the subject body as a velocity within a predetermined velocity range, and generates and outputs a velocity image that indicates the velocity of the moving body based on the calculated velocity and color scale data. The ultrasonic diagnostic apparatus includes an input unit that supplies information indicating an velocity range of interest of the moving body as an input; a velocity range setting control unit that sets a variable detectable velocity range as the predetermined velocity range based on the information supplied from the input unit, the variable detectable velocity range being a wider velocity range than the velocity range of interest and covering the velocity range of interest; and an image processing control unit that allocates the color scale data to each velocity within the detectable velocity range to generate the velocity image based on the color scale data allocated and the calculated velocity.

Further, in the ultrasonic diagnostic apparatus according to the present invention, the velocity range setting control unit sets the detectable velocity range, in which a velocity range that is in a neighborhood of zero and that corresponds to the velocity range of interest is set for removal, as the predetermined velocity range.

Still further, the ultrasonic diagnostic apparatus according to the present invention transmits/receives ultrasound to an interior of a subject body plural times to obtain plural pieces of ultrasound data, generates and outputs an ultrasound tomographic image of the interior of the subject body based on the obtained ultrasound data, calculating a velocity of a moving body that moves in the subject body as a velocity within a predetermined velocity range, and generates and outputs a velocity image that indicates the velocity of the moving body based on the calculated velocity and color scale data. The ultrasonic diagnostic apparatus includes an input unit that supplies information indicating an velocity range of interest of the moving body as an input; and a velocity range setting control unit that sets a variable detectable velocity range as the predetermined velocity range based on the information supplied from the input unit, the variable detectable velocity range being a wider velocity range than the velocity range of interest and covering the velocity range of interest, and the detectable velocity range including a velocity range, which is in a neighborhood of zero and corresponds to the velocity range of interest, for removal.

Still further, the ultrasonic diagnostic apparatus according to the present invention further includes a display unit that displays and outputs plural types of images simultaneously, the images including a color scale image corresponding to the color scale data allocated to each velocity within the detectable velocity range and the velocity image, wherein the image processing control unit controls the display unit to display the plural types of images.

Still further, in the ultrasonic diagnostic apparatus according to the present invention, the image processing control unit controls the display unit to reduce the color scale image corresponding to a velocity out of the velocity range of interest and within the detectable velocity range to a smaller size than a size of the color scale image corresponding to a velocity within the velocity range of interest.

Still further, in the ultrasonic diagnostic apparatus according to the present invention, the image processing control unit, on allocating the color scale data to each velocity within the detectable velocity range, sets hue variation or luminance variation of the color scale data corresponding to variation in velocities within the velocity range of interest larger than the hue variation or the luminance variation of the color scale data corresponding to variation in velocities out of the velocity range of interest and within the detectable velocity range.

Still further, in the ultrasonic diagnostic apparatus according to the present invention, the velocity range setting control unit includes a transmission/reception control unit which sets the velocity range of interest based on the information supplied from the input unit, calculates a reference repetition frequency corresponding to a maximum velocity value within the velocity range of interest and a tentative repetition frequency related with a number of repetitions of transmission and reception of the ultrasound, performs frequency sweeping to sweep the tentative repetition frequency, and sequentially controls the transmission and the reception of the ultrasound using the tentative repetition frequency for each frequency sweeping, and a velocity calculation control unit which sequentially calculates the velocity of the moving body based on the plural pieces of ultrasound data obtained through the control of the transmission and the reception of the ultrasound by the transmission/reception control unit, wherein the transmission/reception control unit sequentially detects the velocities of the moving body from the velocity calculation control unit, performs an aliasing determination to sequentially determine whether the aliasing occurs or not based on the sequentially detected velocities of the moving body, and sets an actual repetition frequency and the detectable velocity range for the control of the transmission and the reception of the ultrasound based on a result of the aliasing determination, and the velocity calculation control unit sets a velocity range to remove a velocity range portion that corresponds to the velocity range of interest and is in a neighborhood of zero from the detectable velocity range, using at least the reference repetition frequency.

EFFECT OF THE INVENTION

The present invention is advantageous in that it realizes an ultrasonic diagnostic apparatus which can suppress the occurrence of aliasing with respect to a detected velocity of a desired moving body in a subject body without compromising a capability to detect a velocity in a desired low velocity range.

Further, the present invention is advantageous in that it realizes an ultrasonic diagnostic apparatus which can suppress the occurrence of aliasing as well as surely display a velocity image, which indicates the velocity of the detected moving body in the subject body, on a screen.

BRIEF DESCRIPTION OF DRAWINGS

FIG. 1 is a block diagram of an exemplary structure of an ultrasonic diagnostic apparatus according to a first embodiment of the present invention;

FIG. 2 is a detailed block diagram of an exemplary structure of a velocity data calculator;

FIG. 3 is a flowchart of a process up to a display of a velocity image of a moving body on a monitor;

FIG. 4 schematically shows one example of an image displayed on the monitor and includes a B mode image and a color Doppler image of an interior of a subject body;

FIG. 5 is a flowchart of a process up to a completion of an actual repetition frequency setting process;

FIG. 6 schematically shows one example of color scale data which is associated with velocities within a detectable velocity range;

FIG. 7 schematically shows an example of variation in luminance in the color scale data against variation in velocity;

FIG. 8 schematically shows another example of variation in luminance in the color scale data against variation in velocity;

FIG. 9 schematically shows one example of variation in hue in the color scale data against variation in velocity;

FIG. 10 schematically shows one example of the color scale data in which a scale of variation in velocities within the velocity range of interest is made larger;

FIG. 11 is a block diagram of one exemplary structure of an ultrasonic diagnostic apparatus according to a second embodiment of the present invention; and

FIG. 12 schematically shows one example of color scale data which is associated with velocities within a velocity range of interest.

EXPLANATIONS OF LETTERS OR NUMERALS

| | |
|---|---|
| 1, 21 | Ultrasonic diagnostic apparatus |
| 2 | Input unit |
| 3 | Ultrasonic transducer |
| 4. | Transmitting/receiving circuit |
| 5 | B mode data calculator |
| 6 | Gray image data generator |
| 7 | Velocity data calculator |
| 8, 22 | Color image data generator |
| 9 | Image synthesizer |
| 10 | Monitor |
| 11 | Storage unit |
| 12 | Control unit |
| 71 | Complex signal generating circuit |
| 72 | Filter |
| 73 | Autocorrelation circuit |
| 74 | Motion information calculator |
| 75 | Threshold processing circuit |
| 100 | Monitor image |
| 101 | B mode image |
| 102 | Color Doppler image |
| 103 | Gray scale |
| 104 | Color scale |
| 110, 120, 130 | Color scale data |
| 110a, 110b, 120a to 120d, 130a, 130b | Color scale element |

BEST MODE(S) FOR CARRYING OUT THE INVENTION

Exemplary embodiments of an.ultrasonic diagnostic apparatus according to the present invention will be described in detail below with reference to the accompanying drawings. It should be noted that the present invention is not limited to the embodiments described below.

First Embodiment

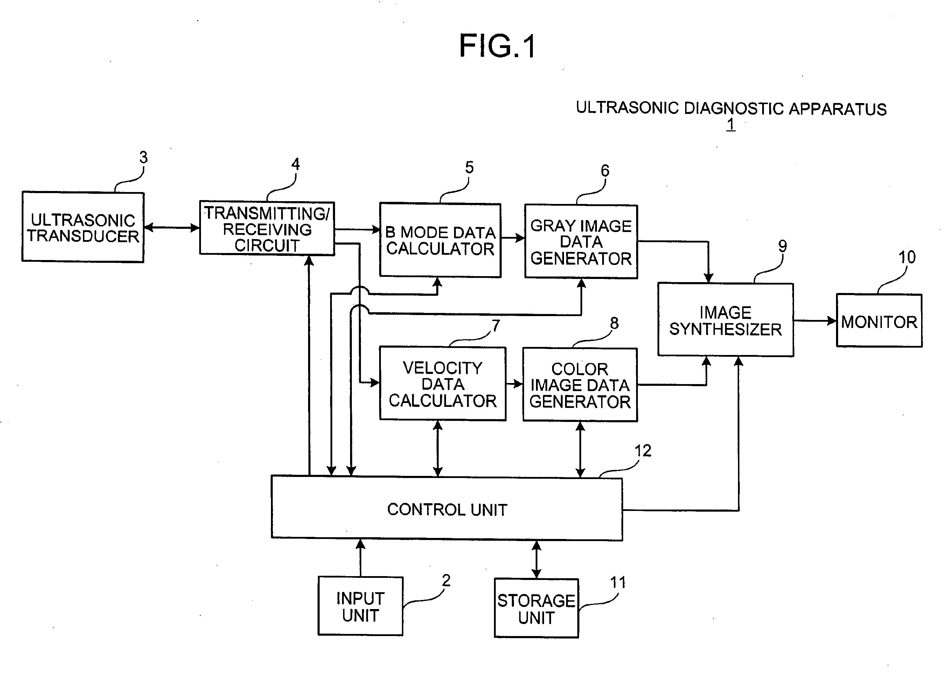

FIG. 1 is a block diagram of one exemplary structure of an ultrasonic diagnostic apparatus according to a first embodiment of the present invention. In FIG. 1, an ultrasonic diagnostic apparatus 1 includes an input unit 2, an ultrasonic transducer 3, a transmitting/receiving circuit 4, a B mode data calculator 5, a gray image data generator 6, a velocity data calculator 7, a color image data generator 8, an image synthesizer 9, a monitor 10, a storage unit 11, and a control unit 12.

The input unit 2 is realized with one of or a combination of a keyboard, a touch panel, a track ball, a mouse, a rotary switch, and the like. The input unit 2 is electrically connected with the control unit 12. The input unit 2 supplies various types of information to the control unit 12 according to a manipulation performed by the operator to input information. The supplied information includes various types of command information for designating starting, ending, switching, and the like of operations performed by respective elements in the ultrasonic diagnostic apparatus 1, various types of parameter information for processing performed by the respective elements in the ultrasonic diagnostic apparatus 1, gray scale information related with gray scale data employed for generation of gray image data, color scale information related with color scale data employed for generation of color image data, and the like.

For example, in response to the information input manipulation by the operator, the input unit 2 supplies operation mode designating information to give command to the control unit 12 to switch the operation mode to one of B mode, color Doppler imaging mode, and tissue Doppler imaging mode. Further, in response to the information input manipulation by the operator, the input unit 2 supplies velocity-range-of-interest designating-information to the control unit 12 to designate a velocity range (velocity range of interest) with respect to a velocity (velocity of interest) of a desired moving body which moves in a region of interest inside the subject body. Here, the B mode is an operation mode in which an ultrasound tomographic image in the subject body, i.e., a B mode image is output and displayed on the monitor 10. The color Doppler imaging mode is a velocity displaying mode in which the velocity of a moving body which moves at relatively high speed, for example the velocity of blood, is detected and displayed as a velocity image. In the color Doppler imaging mode, the velocity image is displayed as a color Doppler image. The tissue Doppler imaging mode is a velocity displaying mode in which the velocity of a moving body which moves at relatively low speed, for example the velocity of a living tissue, is detected and displayed as a velocity image. In the tissue Doppler imaging mode, the velocity image is displayed as a tissue Doppler image. The moving body which moves in the subject body is, for example, blood, living tissue, and ultrasonic contrast agent injected into the subject body, and moves in the subject body relative to the ultrasonic transducer 3.

The ultrasonic transducer 3 is realized with an array transducer in which plural piezoelectric elements of a material such as barium titanate and lead zirconate titanate are arranged. The ultrasonic transducer 3 is electrically connected with the transmitting/receiving circuit 4. The ultrasonic transducer 3 has a function of converting electric pulse signals transmitted from the transmitting/receiving circuit 4 into acoustic pulse signals, i.e., into ultrasound by inverse piezoelectric effect, and a function of converting reflective signals (echo signals) of the acoustic pulse signals obtained through the conversion into electric pulse signals by piezoelectric effect, to output the resulting electric pulse signals to the transmitting/receiving circuit 4. Here, based on the electric pulse signals sequentially transmitted from the transmitting/receiving circuit 4, the ultrasonic transducer 3 sequentially transmits the acoustic pulse signals to the interior of the subject body, for example, sequentially receives the echo signals from the interior of the subject body, and sequentially transmits the electric pulse signals corresponding to the received echo signals to the transmitting/receiving circuit 4. In other words, the ultrasonic transducer 3 repeatedly receives the electric pulse signals from the transmitting/receiving circuit 4 plural times corresponding to each sound ray direction in the subject body, and transmits the acoustic pulse signals the same plural times corresponding to each sound ray direction in the subject body. Then, the ultrasonic transducer 2 receives the echo signals corresponding to the acoustic pulse signals the same plural times. Here, the ultrasonic transducer 3 can perform ultrasonographic scanning plural times for each tomographic plane (each frame) in the subject body under the control of the transmitting/receiving circuit 4.

The transmitting/receiving circuit 4 is realized with a beam forming circuit which controls transmission and reception for sequentially transmitting the electric pulse signals mentioned above to the ultrasonic transducer 3 and sequentially receiving the electric pulse signals after the conversion in the ultrasonic transducer 3. The transmitting/receiving circuit 4 is electrically connected with each of the ultrasonic transducer 3, the B mode data calculator 5, and the velocity data calculator 7. The transmitting/receiving circuit 4 sets a repetition frequency of the electric pulse signal which is repetitiously transmitted plural times in each sound ray direction in the subject body, based on control signals sent from the control unit 12. Further, the transmitting/receiving circuit 4 determines a number of repetitions of transmission of the electric pulse signals for each sound ray direction based on a previously set variable number of repetitions under the control of the control unit 12. The transmitting/receiving circuit 4 repeatedly transmits/receives the electric pulse signals the determined number of repetition times. Thus, the transmitting/receiving circuit 4 can obtain plural pieces of ultrasound data through plural times of ultrasonographic scanning for each frame in the subject body under the control of the control unit 12. Further, the transmitting/receiving circuit 4 transmits the plural pieces of ultrasound data to the B mode data calculator 5 under the control of the control unit 12 when the operation mode of the control unit 12 is the B mode. On the other hand, the transmitting/receiving circuit 4 transmits the plural pieces of ultrasound data alternately to the B mode data calculator 5 and the velocity data calculator 7 for every one frame in the subject body under the control of the control unit 12 when the operation mode of the control unit 12 is the velocity displaying mode.

The transmitting/receiving circuit 4 may perform the transmission/reception of the electric pulse signals in a similar manner as a manner described in Japanese Examined Patent Publication (Kokoku) No. H06-002134. Specifically, the transmitting/receiving circuit 4 may repetitiously transmits/receives the electric pulse signals corresponding to the acoustic pulse signals transmitted/received in the same sound ray direction along with the transmission/reception of the electric pulse signals corresponding to the acoustic pulse signals transmitted/received in a different sound ray direction. Alternatively, the transmitting/receiving circuit 4 may first repetitiously perform the transmission/reception of the electric pulse signals corresponding to the acoustic pulse signals transmitted/received in the same sound ray direction predetermined times, and then goes on to transmit/receive the electric pulse signals corresponding to the acoustic pulse signals transmitted/received in a different sound ray direction.

The B mode data calculator 5 is realized with a known processing circuit that calculates B mode data corresponding to an ultrasound tomographic image (B mode image) in the subject body based on the ultrasound data transmitted from the transmitting/receiving circuit 4. The B mode data calculator 5 is electrically connected with each of the transmitting/receiving circuit 4 and the gray image data generator 6. Specifically, the B mode data calculator 5 performs processing such as band pass filtering, log compression, gain adjustment, contrast adjustment, and frame correlation processing using the plural pieces of ultrasound data sequentially transmitted from the transmitting/receiving circuit 4 corresponding to each frame in the subject body, to calculate the B mode data corresponding to the B mode image for every frame in the subject body under the control of the control unit 12. Here, the B mode data calculator 5 may sequentially calculate the B mode data of each frame in the subject body using the plural pieces of ultrasound data transmitted from the transmitting/receiving circuit 4 for each frame in the subject body. Alternatively, the B mode data calculator 5 may sequentially calculate the B mode data corresponding to plural B mode images arranged in a three-dimensional region in the subject body. The B mode data calculator 5 transmits the obtained B mode data to the gray image data generator 6.

The gray image data generator 6 is realized with a known processing circuit which generates gray image data based on the B mode data transmitted from the B mode data calculator 5, predetermined gray scale data, and a predetermined lookup table. The gray image data generator 6 is electrically connected with each of the B mode data calculator 5 and the image synthesizer 9. Specifically, the gray image data generator 6 sequentially converts the B mode data transmitted from the B mode data calculator 5 into the gray image data using the gray scale data and the lookup table under the control of the control unit 12. The gray image data is image data employed for displaying the B mode image corresponding to the B mode data as a gray image on the monitor 9. The gray image data generator 6 sequentially transmits the gray image data obtained through the conversion to the image synthesizer 9.

The gray image data generator 6 further includes a memory (not shown) such as a Random Access Memory (RAM) and a Read Only Memory (ROM), and stores the gray scale data and the lookup table in an updatable manner. The gray scale data stored in the gray image data generator 6 can be updated to desired gray scale data corresponding to desired gray scale information via the control unit 12, when the operator performs an input manipulation of the desired gray scale information through the input unit 2. The gray scale data is color data, in which different degrees of luminance are assigned to three primary colors (red, green, blue) of light corresponding to the value of the B mode data mentioned above. Properties of colors are changeable corresponding to the B mode data. The gray image data generator 6 can generate the gray image data in a desired luminance corresponding to the B mode data utilizing desired gray scale data.

The velocity data calculator 7 is electrically connected to each of the transmitting/receiving circuit 4 and the color image data generator 8. The velocity data calculator 7 serves to calculate a velocity of the moving body mentioned above based on the ultrasound data sent from the transmitting/receiving circuit 4 and parameter signals transmitted from the control unit 12 under the control of the control unit 12. Specifically, when the operation mode of the control unit 12 is the velocity displaying mode, the velocity data calculator 7 calculates the velocity of the moving body at a spatial position in the subject body for each frame based on the plural pieces of ultrasound data sequentially transmitted from the transmitting/receiving circuit 4 for each frame in the subject body and various parameters based on the parameter signals from the control unit 12 under the control of the control unit 12. Here, the velocity data calculator 7 transmits velocity data (velocity data of interest) corresponding to the velocity of the moving body as the examination target or velocity data (non-target velocity data) corresponding to a velocity of a moving body other than the examination target for each spatial position in the frame in the subject body to the color image data generator 8. A structure of the velocity data calculator 7 will be described later in detail.

The color image data generator 8 is realized with a processing circuit which generates various color image data based on the various types of velocity data sent from the velocity data calculator 7, predetermined color scale data, and a predetermined lookup table. The color image data generator 8 is electrically connected to each of the velocity data calculator 7 and the image synthesizer 9. When the operation mode of the control unit 12 is the velocity displaying mode, the color image data generator 8 sequentially converts the velocity data of interest sent from the velocity data calculator 7 into color image data using the color scale data and the lookup table for each spatial position of each frame in the subject body, and at the same time sequentially converts the non-target velocity data sent from the velocity data calculator 7 into non-target color image data under the control of the control unit 12. The color image data is image data for displaying a velocity image, i.e., a color image, in which the velocity of the moving body is displayed in color when the velocity corresponds to the velocity data of interest, on the monitor 9. On the other hand, the non-target color image data is image data, in which a predetermined color such as a black color is allocated to the velocity of the moving body when the velocity corresponds to the non-target velocity data, and not to be displayed on the monitor 9. The color image data generator 8 sequentially transmits the color image data and the non-target color image data obtained through the conversion to the image synthesizer 9 for each spatial position in each frame in the subject body. The color image data generator 8 transmits the non-target color image data to the image constructing unit 9 as a control signal to prevent the color image of the velocity of the moving body corresponding to the non-target velocity data from being displayed on the monitor 9.

The color image data generator 8 has a memory (not shown) including a RAM, a ROM, or the like, and stores the color scale data and the lookup table in an updatable manner. The color image data generator 8 can store desired color scale data corresponding to desired color scale information in an updatable manner so that the operator can update the color scale data corresponding to the color scale information via the control unit 12 by performing an input manipulation of desired color scale information via the input unit 2. The color scale data is color data consisting of a predetermined combination of luminance or hue of three primary colors (red, green, blue) of the light. The combination is changeable based on the color scale information mentioned above. The color image data generator 8 allocates a certain level of luminance or hue in the color scale data to each velocity within a velocity range (detectable velocity range), using the stored color scale data and the parameter signals sent from the control unit 12. A velocity of a desired moving body in the detectable velocity range can be detected without causing the aliasing mentioned above. The detectable velocity range is wider than the above mentioned velocity range of interest, and at least covers the velocity range of interest. In brief, the color image data generator 8 can allocate a certain level of luminance or hue of the color scale data to each velocity within the velocity range of interest covered by the detectable velocity range, and at the same time, the color image data generator 8 can allocate a yet-allocated level of luminance or hue in the color scale data to each velocity that does not fall within the velocity range of interest though fall within the detectable velocity range, by allocating a certain level of luminance or hue of the color scale data to each velocity within the detectable velocity range based on the parameter signals.

When the operation mode of the control unit 12 is the velocity displaying mode, the image synthesizer 9 synthesizes the gray image data sent from the gray image data generator 6 and the color image data or the non-target color image data sent from the color image data generator 8 with respect to each spatial position of each frame in the subject body, to obtain synthesized image data under the control of the control unit 12. Here, the image synthesizer 9 overwrites the gray image data with the color image data and overwrites the non-target color image data with the gray image data with respect to each spatial position of each frame in the subject body. Thus, the synthesized image data includes the color image data at each spatial position in the subject body corresponding to the color image data and the gray image data at each spatial position not corresponding to the color image data. Thereafter, the image synthesizer 9 converts the obtained synthesized image data into display image data and transmits the display image data to the monitor 10. The monitor 10 is electrically connected to the image synthesizer 9. The monitor 10 displays an ultrasound tomographic image and a velocity image corresponding to the synthesized image data based on the display image data sent from the image synthesizer 9. Thus, the monitor 10 sequentially updates the ultrasound tomographic image and the velocity image corresponding to the synthesized image data for each piece of the display image data sequentially sent from the image synthesizer 9 in real time.

On the other hand, when the operation mode of the control unit 12 is the B mode, the image synthesizer 9 converts the gray image data sent from the gray image data generator 6 into the display image data under the control of the control unit 12, and transmits the display image data to the monitor 10. The monitor 10 displays an ultrasound tomographic image corresponding to the gray image data based on the display image data sent from the image synthesizer 9. updates the ultrasound tomographic image corresponding to the gray image data in real time for each piece of the display image data sequentially sent from the image synthesizer 9.

When the gray scale data is supplied from the gray image data generator 6 directly or via the control unit 12, the image synthesizer 9 converts the gray scale data into the display image data and transmits the display image data to the monitor 10. Then, the monitor 10 displays a gray scale corresponding to the gray scale data based on the display image data sent from the image synthesizer 9. Similarly, when the color scale data is supplied from the color image data generator 8 directly or via the control unit 12, the image synthesizer 9 converts the color scale data into the display image data and transmits the display image data to the monitor 10. Then, the monitor 10 displays a color scale corresponding to the color scale data based on the display image data sent from the image synthesizer 9. Thus, the monitor 10 can display the gray scale and the ultrasound tomographic image on the same screen, or alternatively, the monitor 10 can display the gray scale, the color scale, the ultrasound tomographic image, and the velocity image on the same screen.

The storage unit 11 is realized with various storage unit to which data can be written and from which data can be read out. For example, the storage unit 11 is realized with various types of IC memories such as an EEPROM and a flash memory, a hard disk drive, or a magnetooptical disc drive. The storage unit 11 stores various types of image data such as synthesized image data, gray image data, and color image data supplied from the control unit 12 under the control of the control unit 12. Further, the storage unit 11 stores various pieces of information such as various types of parameter information, gray scale information, and color scale information supplied from the control unit 12 under the control of the control unit 12. Further, the storage unit 11 transmits various pieces of stored information to the control unit 12 under the control of the control unit 12.

The control unit 12 is realized with a ROM in which various types of data such as a processing program is stored in advance, a RAM which temporarily stores operation parameters and the like, and a CPU which executes the processing program. The control unit 12 is electrically connected to the input unit 2, the transmitting/receiving circuit 4, the B mode data calculator 5, the gray image data generator 6, the velocity data calculator 7, the color image data generator 8, the image synthesizer 9, and the storage unit 11. As mentioned above, the control unit 12 controls the operations of the respective elements and input/output of various pieces of information. :

The control unit 12 switches the operation mode to one of the B mode, the color Doppler imaging mode, and the tissue Doppler imaging mode based on operation mode designating information supplied from the input unit 2. Thereafter, the control unit 12 controls the operations and information input/output of the transmitting/receiving circuit 4, the B mode data calculator 5, the gray image data generator 6, the velocity data calculator 7, the color image data generator 8, and the image synthesizer 9 according to the operation mode as described above.

Further, the control unit 12 uniquely sets a velocity range of interest $\pm Vi$ of a desired moving body that moves in a region of interest in the subject body based on the velocity-range-of-interest designating information supplied from the input unit 2, and calculates a reference repetition frequency fi which is a repetition frequency adopted when the velocity range of interest $\pm Vi$ is set as a velocity range from which a velocity of interest can be detected. Here, the velocity range of interest $\pm Vi$ is defined as a velocity range covering a range from a minimum velocity -Vi to a maximum velocity Vi. Thereafter, the control unit 12 sets an actual repetition frequency fr for controlling the number of repetitions of transmission/reception of the electric pulse signals by the transmitting/receiving circuit 4 based on the reference repetition frequency fi, and transmits a control signal corresponding to the actual repetition frequency fr to the transmitting/ receiving circuit 4. Here, the transmitting/receiving circuit 4 sets the repetition frequency of the electric pulse signals based on the control signal sent from the control unit 12. Further, the transmitting/receiving circuit 4 determines the number of repetitions of transmission/reception of the electric pulse signals based on the previously set variable number of repetitions.

Further, the control unit 12 sets a detectable velocity range $\pm Vr$ of a desired moving body that moves in the region of interest in the subject body based at least on the velocity range of interest $\pm Vi$. Here, the control unit 12 sets the detectable velocity range $\pm Vr$ as a variable range relative to the velocity range of interest $\pm Vi$. Here, the detectable velocity range $\pm Vr$ is defined as a velocity range covering a range from a minimum velocity -Vr to a maximum velocity Vr. Further, the control unit 12 calculates a cutoff frequency fc for removing a velocity component of a non-target moving body that moves within the region of interest in the subject body. Thereafter, the control unit 12 transmits each parameter signal corresponding to the actual repetition frequency fr and the cutoff frequency fc to the velocity data calculator 7. Still further, the control unit 12 transmits each parameter signal corresponding to the velocity range of interest $\pm Vi$ and the detectable velocity range $\pm Vr$ to the color image data generator 8.

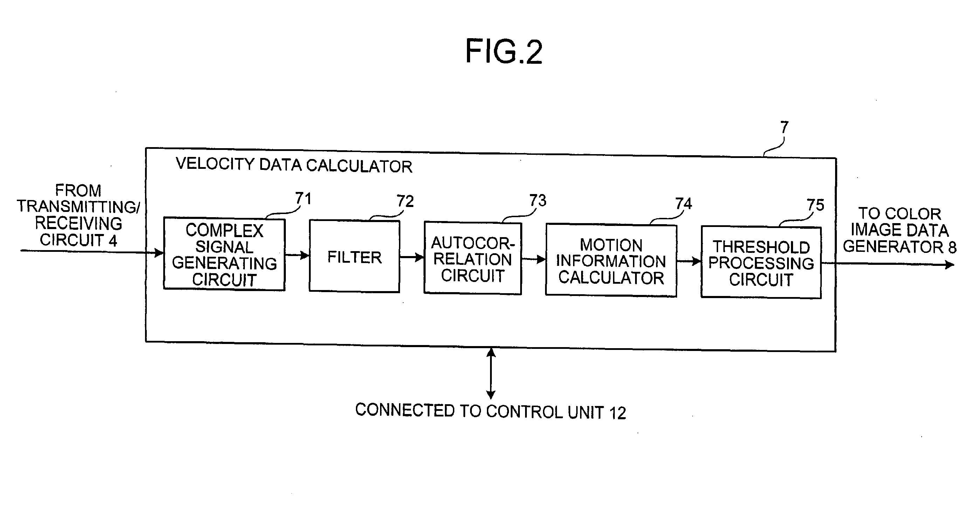

A structure of the velocity data calculator 7 will be described in detail. FIG. 2 is a detailed block diagram of the structure of the velocity data calculator 7. In FIG. 2, the velocity data calculator 7 includes a complex signal generating circuit 71, a filter 72, an autocorrelation circuit 73, a motion information calculator 74, and a threshold processing circuit 75.

The complex signal generating circuit 71 is realized with a quadrature detector, and serves to convert the electric pulse signals that correspond to the ultrasound data and are sent from the transmitting/receiving circuit 4 into complex signals. Specifically, the complex signal generating circuit 71 performs a multiplication process using a sinusoidal signal and the electric pulse signal transmitted from the transmitting/receiving circuit 4 to obtain an electric signal. Here, a phase of the sinusoidal signal is different from a phase of the electric pulse signal by 90°. Then, the complex signal generating circuit 71 lets the obtained electric signal pass through a low pass filter, thereby obtaining the complex signal. Thereafter, the complex signal generating circuit 71 transmits the resulting complex signal to the filter 72.

For example, when the operation mode of the control unit 12 is the color Doppler imaging mode or the tissue Doppler imaging mode, the transmitting/receiving circuit 4 repeats the transmission/reception of the electric pulse signals the number of repetition times mentioned above (for example, approximately eight times) for every sound ray direction in which the desired moving body is detected. Here, the complex signal generating circuit 71 receives groups of electric pulse signals corresponding to groups of ultrasound data of the same number (eight, for example) as the number of repetitions in every sound ray direction, in which the desired moving body is detected, from the transmitting/receiving circuit 4. At the same time, the complex signal generating circuit 71 obtains groups of complex signals resulting from a conversion of the groups of electric pulse signals. Thus, the complex signal generating circuit 71 obtains the groups of complex signals corresponding to the groups of ultrasound data obtained as a result of detection of the desired moving body in each position for a two-dimensional space or a three-dimensional space in the region of interest in the subject body. Then, the complex signal generating circuit 71 transmits the obtained groups of complex signals to the filter 72.

The complex signal generating circuit 71 may include a memory (not shown) such as a RAM, and store the obtained groups of complex signals. Further, the complex signal generating circuit 71 may transmit the obtained groups of complex signals to the control unit 12 and the control unit 12 may store and manage the groups of complex signals.

The filter 72 is realized with a digital Finite Impulse Response (FIR) filter or a digital Infinite Impulse Response (IIR) filter in which a Digital Signal Processor (DSP), a Field Programmable Gate Array (FPGA), or the like is provided. The filter 72 performs a filtering process on each of a group of real number signals and a group of imaginary number signals of the groups of complex signals sequentially transmitted from the complex signal generating circuit 71 under the control of the control unit 12.

For example, when the operation mode of the control unit 12 is the color Doppler imaging mode, the control unit 12 transmits the parameter signal corresponding to the cutoff frequency fc mentioned above to the filter 72. The filter 72 receives the parameter signal from the control unit 12, and at the same time, sets the cutoff frequency fc for the filtering process based on the received parameter signal. Here, when the velocity of the moving body, such as blood, that moves at relatively high speed is to be detected, the filter 72 serves as a known MTI filter to perform a filtering process on the group of complex signals, and removes low frequency components, i.e., components with a small variation, as noises from the group of complex signals. This process is equivalent to the removal of components corresponding to the velocity of the moving body which moves at relatively low speed from the group of complex signals. Thereafter, the filter 72 transmits the group of complex signals that includes the group of real number signals and the group of imaginary number signals subjected to the filtering process for removal of the low frequency components to the autocorrelation circuit 73.

On the other hand, when the operation mode of the control unit 12 is the tissue Doppler imaging mode, the filter 72 sets a predetermined filter coefficient under the control of the control unit 12, and at the same time serves as a known low pass filter to perform a filtering process on the group of complex signals when the velocity of the moving body, such as living tissue, that moves at relatively low speed is to be detected. Here, the filter 72 removes high frequency components, i.e., components with large variations as noises from the group of complex signals. This process is equivalent to the removal of components corresponding to the velocity of the moving body that moves at relatively high speed from the group of complex signals. Thereafter, the filter 72 transmits the group of complex signals that includes the group of real number signals and the group of imaginary number signals subjected to the filtering process for the removal of the low frequency components to the autocorrelation circuit 73. When the operation mode of the control unit 12 is the tissue Doppler imaging mode, the filter 72 may stop serving as the filter under the control of the control unit 12. Then, the filter 72 does not perform the filtering process on the group of complex signals sent from the complex signal generating circuit 71 and transmits the group of complex signals as it is to the autocorrelation circuit 73.

The autocorrelation circuit 73 is realized with a DSP, a FPGA, or the like. The autocorrelation circuit 73 calculates a complex autocorrelation value R of the group of complex signals based on the group of complex signals sent from the filter 72. For example, a complex number $Z_a$, which indicates $a^{th}$ complex signal among N (here, N is an integer equal to or larger than two) complex signals in the group of complex signals, is represented by the following expression (1):

$$Z_a = x_a + jy_a \quad (a = 1 \sim N) \tag{1}$$

The autocorrelation circuit 73 calculates the complex autocorrelation value R of the group of complex signals based on the following expression (2):

$$R = \sum_{a=1}^{N-1} Z_{a+1} \times Z_a * \tag{2}$$

In expression (2), complex number $Z_a^*$ is a complex number which is conjugate with the complex number $Z_a$. The autocorrelation circuit 73 supplies an electric signal corresponding to the complex autocorrelation value R calculated based on expression (2) to the motion information calculator 74.

The motion information calculator 74 is realized with a DSP, a FPGA, or the like. The motion information calculator 74 calculates a velocity V of a desired moving body and an echo intensity I at each spatial position of every frame in the subject body under the control of the control unit 12. Specifically, the motion information calculator 74 calculates the velocity V using the complex autocorrelation value R based on electric signals sent from the autocorrelation circuit 73, the actual repetition frequency fr based on parameter signals sent from the control unit 12, a sound speed c, and a central frequency f0 of electric pulse signals transmitted/received to/from the transmitting/receiving circuit 4, and based on the following expression (3). Further, the motion information calculator 74 calculates the echo intensity I based on the following expression (4).

$$V = \frac{c}{4\pi \times f_0 \times T} \times \tan^{-1}\left(\frac{Ry}{Rx}\right) \tag{3}$$

$$I = |R| \tag{4}$$

According to expression (3), the motion information calculator 74 obtains a real number component Rx and an imaginary number component Ry of the complex autocorrelation value R. Further, the motion information calculator 74 obtains frequency T as an inverse number of the actual repetition frequency fr. Here, the frequency T is an operation cycle of repetitious transmission/reception of electric pulse signals by the transmitting/receiving circuit 4 for each sound ray direction in the subject body.

Thereafter, the motion information calculator 74 supplies electric signals corresponding to the velocity V, which is calculated based on the expression (3) and electric signals corresponding to the echo intensity I calculated based on the expression (4) for each spatial position of each frame in the subject body to the threshold processing circuit 75. Further, the motion information calculator 74 transmits the electric signals corresponding to the velocity V calculated based on the expression (3) to the control unit 12 under the control of the control unit 12. The control unit 12 can detect the velocity V calculated by the motion information calculator 74 with respect to the moving body in real time.

Here, the motion information calculator 74 may include a memory (not shown) such as a RAM, and may store operation parameters such as the sound speed c and the central frequency $f_0$ in advance. Further, the motion information calculator 74 may obtain the operation parameters such as the sound speed c and the central frequency $f_0$ based on the parameter signals sent from the control unit 12.

The threshold processing circuit 75 is realized with a DSP, a FPGA, or the like. The threshold processing circuit 75 performs a display determination process, in which the threshold processing circuit 75 determines whether the velocity V calculated by the motion information calculator 74 with respect to the moving body is a velocity to be displayed on the monitor 10 with respect to the moving body or not, under the control of the control unit 12. To perform the display determination process, the threshold processing circuit 75 obtains each of the velocity V and the echo intensity I at each spatial position of every frame in the subject body based on the respective electric signals sent from the motion information calculator 74, and compares the obtained velocity V with a predetermined velocity threshold and compares the obtained echo intensity I and a predetermined intensity threshold of the echo intensity.

For example, when the operation mode of the control unit 12 is the color Doppler imaging mode, the threshold processing circuit 75 compares the velocity V of each spatial position of every frame in the subject body with velocity threshold $V_{TH1}$, and determines whether the velocity V satisfies the following expression (5) or not:

$$|V| > V_{TH1} \tag{5}$$

At the same time, the threshold processing circuit 75 compares the echo intensity I at each spatial position of every frame in the subject body with the intensity threshold $I_{TH1}$, $I_{TH2}$ under the control of the control unit 12, and determines whether the echo intensity I satisfies the following expression (6):

$$I_{TH1} < I < I_{TH2} \qquad\qquad (6)$$

Here, the velocity threshold $V_{TH1}$ is a threshold for the threshold processing unit 75 to determine whether the velocity V is a velocity of a moving body, such as blood, that moves at relatively high speed or not. The intensity threshold $I_{TH1}$ is a threshold for the threshold processing unit 75 to determine whether the obtained echo intensity I represents a noise or not. The intensity threshold $I_{TH2}$ is a threshold for the threshold processing unit 75 to determine whether a moving body that moves at the velocity V is a solid such as a living tissue or a fluid such as blood.

Here, the threshold processing circuit 75 determines that the velocity V that satisfies expression (5) is the velocity of the moving body that moves at relatively high speed, i.e., the moving body as the examination target. Further, the threshold processing circuit 75 determines that the echo intensity I represents a noise when the echo intensity I is equal to or lower than the intensity threshold $I_{TH1}$. Further, the threshold processing circuit 75 determines that the velocity V corresponding to the echo intensity I which is below the intensity threshold $I_{TH2}$ is the velocity of a fluid such as blood.

Further, the threshold processing circuit 75 determines that the velocity V corresponding to the echo intensity I which is above the intensity threshold $I_{TH2}$ is the velocity of a solid such as a living tissue. The threshold processing circuit 75 determines that the velocity V that satisfies the expression (5) and that corresponds to the echo intensity I that satisfies expression (6) is the velocity of a desired moving body whose image is to be displayed on the monitor 10 as the velocity image in the color Doppler imaging mode. Thus, the threshold processing circuit 75 can determine whether the velocity V at each spatial position of every frame in the subject body is a velocity of a desired moving body, such as blood, to be displayed on the monitor 10 as the velocity image or not. Thereafter, the threshold processing circuit 75 transmits the velocity V that satisfies expression (5) and that corresponds to the echo intensity I that satisfies expression (6) as the above mentioned velocity data of interest to the color image data generator 8 for each spatial position of every frame in the subject body. On the other hand, the threshold processing circuit 75 transmits the velocity V other than the velocity V that satisfies expression (5) and that corresponds to the echo intensity I that satisfies expression (6) to the color image data generator 8 as the above mentioned non-target velocity data. Here, the threshold processing circuit 75 may replace the velocity V that does not satisfy expression (5) or that corresponds to the echo intensity I that does not satisfy expression (6) with the zero velocity, and may transmit the data of the zero velocity to the color image data generator 8 as the non-target velocity data.

Here, an optimal value for each of the velocity threshold $V_{TH1}$ and the intensity thresholds $I_{TH1}$, $I_{TH2}$ can be obtained experimentally. Further, the threshold processing circuit 75 can perform the display determination process on the moving body other than blood in a similar manner by setting the velocity threshold $V_{TH1}$ or the intensity thresholds $I_{TH1}$, $I_{TH2}$ to appropriate values. Further, the threshold processing circuit 75 may include a memory (not shown) such as a RAM, and may store the velocity threshold $V_{TH1}$ or the intensity thresholds $I_{TH1}$, $I_{TH2}$ in advance. Further, the threshold processing circuit 75 may obtain the velocity threshold $V_{TH1}$, or the intensity thresholds $I_{TH1}$, $I_{TH2}$, based on the parameter signals sent from the control unit 12.

On the other hand, when the operation mode of the control unit 12 is the tissue Doppler imaging mode, the threshold processing circuit 75 compares the velocity V at each spatial position of every frame in the subject body with the velocity threshold $V_{TH2}$ under the control of the control unit 12, and determines whether the velocity V satisfies the following expression (7) or not:

$$|V| < V_{TH2} \qquad\qquad (7)$$

At the same time, the threshold processing circuit 75 compares the echo intensity I at each spatial position of every frame in the subject body with the intensity threshold $I_{TH3}$ under the control of the control unit 12, and determines whether the echo intensity I satisfies the following expression (8) or not:

$$I > I_{TH3} \qquad\qquad (8)$$

Here, the velocity threshold $V_{TH2}$ is a threshold for the threshold processing circuit 75 to determine whether the velocity V is a velocity of a moving body, such as a living tissue, that moves at relatively low speed or not. The intensity threshold $I_{TH3}$ is a threshold for the threshold processing circuit 75 to determine whether a moving body that moves at the velocity V is a solid such as a living tissue or not.

Here, the threshold processing circuit 75 determines that the velocity V that satisfies expression (7) as a velocity of a moving body that moves at a relatively low speed, i.e., a velocity of an examination target. Further, the threshold processing circuit 75 determines that the velocity V corresponding to the echo intensity I that is higher than the intensity threshold $I_{TH3}$ is the velocity of a solid such as a living tissue. Therefore, the threshold processing circuit 75 determines that the velocity V that satisfies expression (7) and that corresponds to the echo intensity I that satisfies expression (8) is a velocity of a desired moving body which is to be displayed on the monitor 10 as the velocity image in the tissue Doppler imaging mode. Thus, the threshold processing circuit 75 can determine whether the velocity V at each spatial position of every frame in the subject body is a velocity of a desired moving body, such as a living tissue, to be displayed on the monitor 10 as the velocity image. Thereafter, the threshold processing circuit 75 transmits the velocity V that satisfies expression (7) and that corresponds to the echo intensity I that satisfies expression (8) as the above mentioned velocity data of interest to the color image data generator 8 for each spatial position of every frame in the subject body. Further, the threshold processing circuit 75 transmits the velocity V other than the velocity V that satisfies expression (7) and that corresponds to the echo intensity I that satisfies expression (.8) as the above mentioned non-target velocity data to the color image data generator 8. Here, the threshold processing circuit 75 may replace the velocity V that does not satisfy expression (7) or the velocity V corresponding to the echo intensity I that does not satisfy expression (8) with the zero velocity, and may transmit the data of zero velocity to the color image data generator 8 as the non-target velocity data.

An optimal value of each of the velocity threshold $V_{TH2}$ and the intensity threshold $I_{TH3}$ can be obtained experimentally. Further, the threshold processing circuit 75 can perform the display determination process on a moving body other than a living tissue in a similar manner by setting the velocity threshold or the intensity threshold to an appropriate value. Further, the threshold processing circuit 75 may store the velocity threshold $V_{TH2}$ or the intensity threshold $I_{TH3}$ in advance. Alternatively, the threshold processing circuit 75 may obtain the velocity threshold $V_{TH2}$ or the intensity threshold $I_{TH3}$ based on the parameter signals sent from the control unit 12.

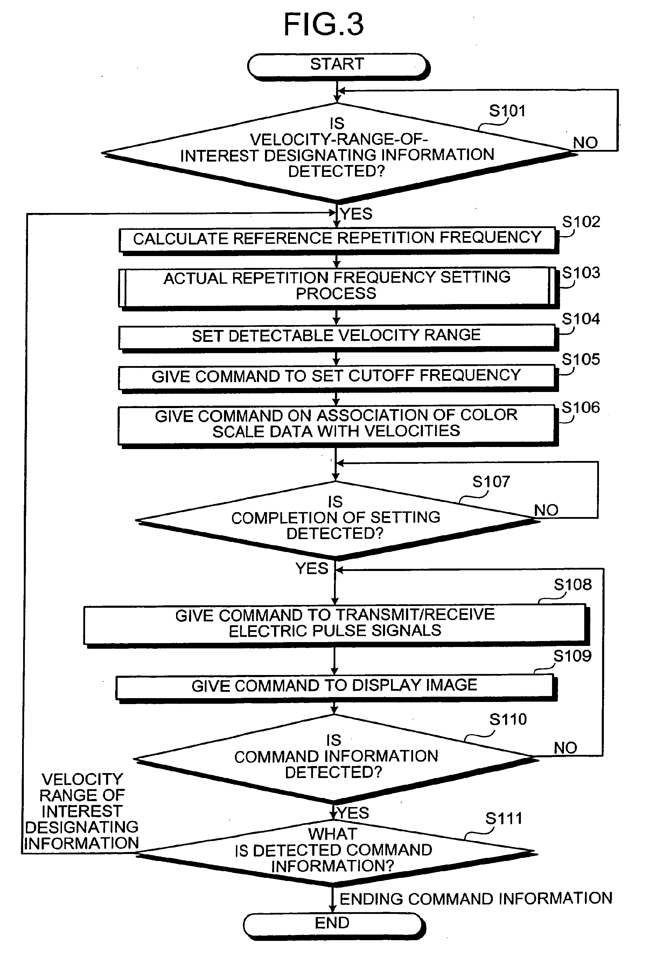

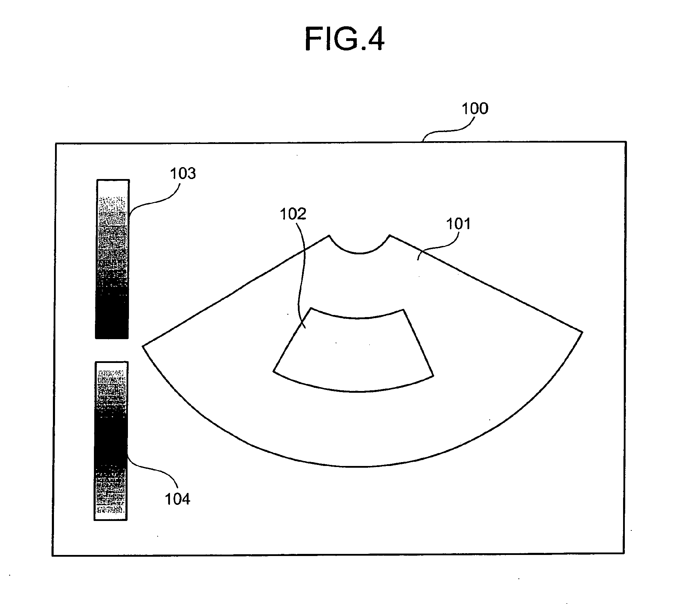

A process in the control unit 12 in the color Doppler imaging mode up to the display/output of a velocity image, i.e., a color Doppler image, that indicates the velocity of a moving body in the subject body will be described in detail. FIG. 3 is a flowchart illustrating the process in the control unit up to the display/output of the velocity image of the moving body in the subject body on the monitor 10. FIG. 4 is a schematic diagram of an example of an image displayed on the monitor including a B mode image and a color Doppler image of.the interior of the subject body.

As shown in FIG. 3, the operator first manipulates the input unit 2 to select the above mentioned velocity range of interest with respect to a desired moving body that moves within the subject body. In the input unit 2, a desired number of options are set to be selected as the velocity range of interest. The options are selected according to a type of the ultrasonic transducer 3, an observed region of the subject body, a frequency of transmitted/received acoustic pulse signals, and the like. The operator manipulates the input unit 2 to select the desired velocity range of interest from options set in the input unit 2, for example, the operator selects one of options indicating the velocity such as 5 cm/s, 10 cm/s, 20 cm/s, and 40 cm/s as the velocity range of interest. Then, the input unit 2 supplies the velocity-range-of-interest designating information which indicates the velocity range of interest selected by the operator to the control unit 12. The control unit 12 detects the velocity-range-of-interest designating information supplied from the input unit 2 (Yes in step S101), and sets the above mentioned velocity range of interest $\pm$Vi based on the detected velocity-range-of-interest designating information. At the same time, the control unit 12 calculates the reference repetition frequency fi mentioned above based on the following expression (9) (step 5102):

$$ fi = \frac{4 \times f_0 \times Vi}{c} \tag{9} $$

In expression (9), the maximum velocity Vi is the maximum velocity within the velocity range of interest $\pm$Vi.

On the other hand, if the operator does not manipulate the input unit 2 to select the velocity range of interest, the control unit 12 does not detect the velocity-range-of-interest designating information (No in step S101) and repeats the process of step S101.

Then, the control unit 12 performs an actual repetition frequency setting process to set the above mentioned actual repetition frequency fr using the reference repetition frequency fi calculated in step S102 and a variable coefficient parameter $\alpha$ (here, $\alpha$ is a real number equal to or larger than 1) previously set (step S103). The control unit 12 obtains the actual repetition frequency fr based on the following expression (10), and transmits parameter signals indicating the obtained actual repetition frequency fr to the motion information calculator 74 as mentioned above.

$$fr = \alpha \times fi \qquad (10)$$

Thereafter, the control unit 12 calculates the maximum velocity Vr within the detectable velocity range ±Vr mentioned above using the actual repetition frequency fr set in step S103, the central frequency $f_0$, and the sound speed c mentioned above. Then, the control unit 12 sets the detectable velocity range ±Vr based on the calculated maximum velocity Vr and a minimum velocity -Vr which is obtained by inverting the sign of the maximum velocity Vr (step S104). Since the maximum velocity Vi within the velocity range of interest ±Vi set by the control unit 12 is represented by the following expression (11) based on expression (9), the control unit 12 can calculate the maximum velocity Vr based on the following expression (12):
$$Vi = \frac{c \times fi}{4 \times f_0} \qquad (11)$$

$$Vr = \frac{c \times fr}{4 \times f_0} = \alpha \times Vi \qquad (12)$$

When the control unit 12 obtains the velocity range of interest ±Vi and the detectable velocity range ±Vr, the control unit 12 calculates the cutoff frequency fc based on the following expression (13) using the maximum velocity Vi within the velocity range of interest ±Vi, the maximum velocity Vr within the detectable velocity range ±Vr, and a coefficient parameter β (here, β is a positive decimal number) set in advance. At the same time, the control unit 12 gives a command to the filter 72 to set the cutoff frequency fc by transmitting the parameter signals indicating the calculated cutoff frequency fc to the filter 72 (step S105). Here, the filter 72 sets the cutoff frequency fc as a cutoff frequency for filtering process and comes to serve as a MTI filter mentioned above.
$$fc = \beta \times fi \times \frac{Vi}{Vr} = \frac{\beta \times fi}{\alpha} \qquad (13)$$

The coefficient parameter β is set in a variable manner depending on the moving body whose velocity is to be detected. The control unit 12 sets the coefficient parameter β in a variable manner in response to the manipulation of the input unit 2 by the operator. For example, the coefficient parameter β is desirably set to a value approximately within a range of 0.1 to 0.2 when the moving body whose velocity is to be detected is a moving body, such as blood, that moves at relatively high speed.
Thereafter, the control unit 12 gives a command to the color image data generator 8 on association between the color scale data mentioned above and the velocities by transmitting the parameter signals indicating the velocity range of interest ±Vi and the detectable velocity range ±Vr to the color image data generator 8 (step S106). Here, the color image data generator 8 allocates a certain level of luminance or hue of the color scale data to each velocity within the velocity range of interest ±Vi using the velocity range of interest ±Vi and the detectable velocity range ±Vr based on the parameter signals and the stored color scale data. At the same time, the color image data generator 8 allocates a yet-allocated level of the luminance or the hue in the color scale data to each velocity out of the velocity range of interest ±Vi though within the detectable velocity range ±Vr. Thus, the color image data generator 8 finishes associating the velocity with the color scale data.
When the parameter signals indicating the cutoff frequency fc is transmitted to the filter 72 and the parameter signals each indicating the velocity range of interest ±Vi and the detectable velocity range ±Vr are transmitted to the color image data generator 8, the control unit 12 confirms whether the setting of the cutoff frequency fc by the filter 72 in step S105 and the setting of the association of the color scale data with the velocity by the color image data generator 8 in step S106 are finished or not. The control unit 12 confirms the completion of the setting of the cutoff frequency fc based on response signals indicating the completion of the setting of the cutoff frequency fc by the filter 72, and confirms the completion of the setting of the association of the color scale data with the velocity based on response signals indicating the completion of the association between the color scale data and the velocity by the color image data generator 8. When the control unit 12 does not receive the response signals from the filter 72 or the response signals from the color image data generator 8, the control unit 12 does not detect either of the completion of setting of the cutoff frequency fc or the completion of setting of the association between the color scale data and the velocity (No in step S107), and repeats the process of step S107.

On the other hand, when the control unit 12 receives the response signals from the filter 72 and the response signals from the color image data generator 8, the control unit 12 detects the completion of setting of the cutoff frequency fc and the completion of setting of the association between the color scale data and the velocity (Yes in step S107). Then, the control unit 12 gives a command to the transmitting/receiving circuit 4 to transmit/receive the electric pulse signals mentioned above by transmitting control signals indicating the actual repetition frequency fr set in step S103 to the transmitting/receiving circuit 4 (step S108). Then, the transmitting/receiving circuit 4 transmits/receives the electric pulse signals a number of times determined by the actual repetition frequency fr in a repetitious manner.

Then, the control unit 12 gives a command to the image synthesizer 9 to display a monitor image which includes at least a color Doppler image indicating the velocity V calculated by the velocity data calculator 7 with respect to the moving body and a B mode image of the interior of the subject body on the monitor 10 (step S109). Here, the image synthesizer 9 generates synthesized image data mentioned above, converts the synthesized image data into display image data, and transmits the display image data to the monitor 10 under the control of the control unit 12. The monitor 10 displays/outputs a monitor image 100 illustrated in FIG. 4 based on the display image data sent from the image synthesizer 9. For example, the monitor 10, as shown in FIG. 4, displays/outputs the monitor image 100 which includes a B mode image 101 indicating the interior of the subject body, a color Doppler image 102 of a moving body that moves in a desired region, i.e., a region of interest in the subject body, a gray scale 103 of the B mode image 101, and a color scale 104 of the color Doppler image 102. The operator can grasp the velocity, e.g., the flow rate, and the orientation of the desired moving body, such as blood, that moves at relatively high speed within the subject body by referring to the color Doppler image 102 and the color scale 104. The operator can further set a displayed region of the color Doppler image 102 on the B mode image 101 as a desired region by manipulating the input unit 2.

If the operator does not manipulate the input unit 2 to input ending command information or velocity-range-of-interest designating information, the controlling uni12 does not detect these command information (No in step S110), and repeats the process from step S108. Here, the ending command information serves to give command to end the detection of the velocity of the desired moving body. For example, the ending command information serves to give command to the transmitting/receiving circuit 4 to end the transmission/reception of the electric pulse signals mentioned above.

On the other hand, when the control unit 12 detects the command information supplied from the input unit 2 (Yes in step S110) and the detected command information is the velocity-range-of-interest designating information as mentioned above (step S111; velocity-range-of-interest designating information), the control unit 12 repeats the process from step S102. Further, when the control unit 12 detects the command information supplied from the input unit 12 (Yes in step S110), and the detected command information is the ending command information as mentioned above (step S111; ending command information), the control unit 12 gives command to the transmitting/receiving circuit 4 to end the transmission/reception of the electric pulse signals mentioned above, thereby ending various types of processes related with the detection of the velocity of the desired moving body.

Here, the control unit 12 can display/output the velocity image that indicates the velocity of the moving body inside the subject body, i.e., the tissue Doppler image, on the monitor 10 by performing the process from step S101 to step S111 in the tissue Doppler imaging mode. The control unit 12, then, performs a process to set a predetermined filter coefficient in the filter 72 and allows the filter 72 to serve as a low pass filter, or the control unit 12 performs a process to stop the filter 72 from working as a filter instead of performing the process of step S105 mentioned above. Then, the monitor 10 displays/outputs the tissue Doppler image instead of the color Doppler image 102 of the monitor image 100 shown in FIG. 4; and at the same time, displays/outputs a color scale of the tissue Doppler image instead of the color scale 104. The operator can grasp the velocity of the desired moving body that moves at relatively low speed in the subject body, for example, a velocity of a motion of a living tissue, by referring to the tissue Doppler image and the color scale thereof.

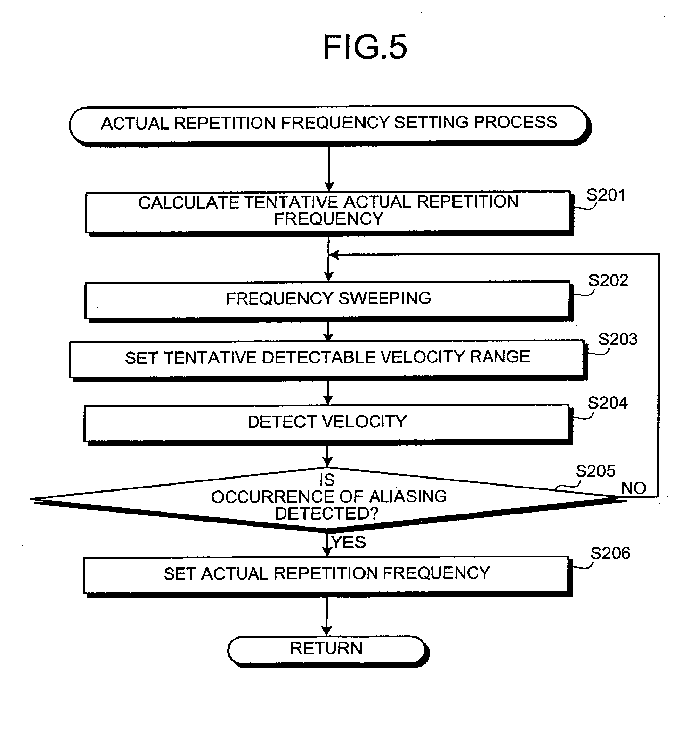

A process of the control unit 12 up to the completion of the setting of the actual repetition frequency in step S103 will be described in detail. FIG. 5 is a flowchart of the process up to the completion of the actual repetition frequency setting process in step S103. As shown in FIG. 5, when the control unit 12 calculates the reference repetition frequency fi in step S102, the control unit 12 proceeds to calculate a tentative actual repetition frequency fr' by multiplying the obtained reference repetition frequency fi and a coefficient parameter $\alpha_{max}$ which is a maximum value of the variable coefficient parameter $\alpha$ (step S201), similarly to expression (10).

Thereafter, the control unit 12 gradually brings the obtained tentative actual repetition frequency fr' close to the reference repetition frequency fi, and transmits control signals indicating the obtained tentative actual repetition frequency fr' to the transmitting/receiving circuit 4, thereby performing the frequency sweeping to control the transmission/reception of the electric pulse signals by the transmitting/receiving circuit 4 (step S202). Here, the control unit 12 varies the coefficient parameter $\alpha$ which is multiplied with the reference repetition frequency fi from the maximum value (i.e., $\alpha = \alpha_{max}$) to one ($\alpha = 1$) at predetermined numerical intervals, thereby sequentially varying the tentative actual repetition frequency fr'.

Further, at every frequency sweeping of step S202, the control unit 12 calculates a tentative maximum velocity Vr' which is a maximum value within the detectable velocity range and corresponds to the tentative actual repetition frequency fr' in a similar manner to the process in step S104, and sets a tentative detectable velocity range $\pm$Vr' based on the obtained tentative maximum velocity Vr' (step S203).

Here, if the operation mode is the color Doppler imaging mode, every time the tentative detectable velocity range $\pm$Vr' is set, the control unit 12 may tentatively set the cutoff frequency fc in the filter 72 by performing a process substantially the same as the procedure of step S105. Further, if the operation mode is the tissue Doppler imaging mode, the control unit 12 controls the filter 72 to stop the filter 72 from serving as a filter when the tentative detectable velocity range $\pm$Vr' is set.

On the other hand, when the control unit 12 transmits the control signals indicating the tentative actual repetition frequency fr' to the transmitting/receiving circuit 4, the transmitting/receiving circuit 4 transmits/receives the electric pulse signals a number of times based on the tentative actual repetition frequency fr' in a repetitious manner under the control of the control unit 12. Here, the motion information calculator 74 calculates the velocity V of a moving body in the subject body based on the group of ultrasound data obtained through repetitious transmission/reception of the electric pulse signals the number of times based on the tentative actual repetition frequency fr' as mentioned above. The control unit 12 controls the motion information calculator 74 so as to feed back the calculated velocity V, and detects the calculated velocity V from the motion information calculator 74 (step S204).

Thereafter, the control unit 12 determines whether the aliasing occurs within the tentative detectable velocity range $\pm$Vr' set in step S203 using the velocity V detected from the motion information calculator 74. Here, the control unit 12 determines whether the aliasing occurs or not by detecting whether the code of the velocity V is inverted or not. When the coefficient parameter $\alpha$ is in the neighborhood of the maximum value (= $\alpha_{max}$) in the frequency sweeping of step S202, the tentative actual repetition frequency fr' based on the coefficient parameter $\alpha$ is sufficiently larger than the reference repetition frequency fi. Therefore, the tentative detectable velocity range $\pm$Vr' based on the tentative actual repetition frequency fr' has a sufficiently wider velocity range, i.e., velocity width, than the velocity range of interest $\pm$Vi. Here, the velocity of the moving body is assumed to be the velocity within the velocity range of interest $\pm$Vi, and is considered to be within the tentative detectable velocity range $\pm$Vr'. Therefore, the control unit 12 can detect the velocity V calculated by the motion information calculator 74 as a velocity with a correct sign based on the sampling theorem.

The control unit 12 confirms whether the sign of the velocity V is inverted for each of the frequency sweeping of step S202 with respect to the velocity V detected as the velocity with the correct sign. When the inversion of the sign of the velocity V is not confirmed, the control unit 12 does not detect the aliasing within the tentative detectable velocity range $\pm$Vr' (No in step S205), and repeats the process after step S202. On the other hand, when the inversion of the sign of the velocity V is confirmed, the control unit 12 detects the occurrence of the aliasing within the tentative detectable velocity range $\pm$Vr' (Yes in step S205), and sets the tentative actual repetition frequency fr', which is obtained by multiplying the coefficient parameter $\alpha$ set in the last of the frequency sweeping during which the aliasing is not detected, and the reference repetition frequency fi as the actual repetition frequency fr (step S206).

In place of step S201 described above, the control unit 12 may calculate the tentative actual repetition frequency fr' by multiplying the reference repetition frequency fi and the minimum value (i.e., 1) of the variable coefficient parameter $\alpha$, similarly to expression (10). Further, in place of step S202 described above, the control unit 12 may gradually increase the tentative actual repetition frequency fr' and transmit the control signals indicating the tentative actual repetition frequency fr' to the transmitting/receiving circuit 4, thereby performing the frequency sweeping to control the transmission/reception of the electric pulse signals by the transmitting/receiving circuit 4. In brief, the control unit 12 may gradually increase the coefficient parameter $\alpha$ which is multiplied with the reference repetition frequency fi from the minimum value (i.e., $\alpha = 1$) at predetermined numerical intervals, thereby sequentially varying the tentative actual repetition frequency fr'.

Then, the control unit 12 detects the velocity V from the motion information calculator 74. The sign of the velocity V here is likely to have been inverted, if the coefficient parameter $\alpha$, is in the neighborhood of the minimum value. Thus, when the control unit 12 confirms the inversion of the sign of the velocity V detected from the motion information calculator 74, in other words, when the occurrence of the aliasing is detected, the control unit 12 repeats the process from the frequency sweeping, whereas when the control unit ceases to confirm the inversion of the sign of the velocity V, i.e., when the occurrence of the aliasing ceases to be detected, the control unit 12 sets the actual repetition frequency fr, in place of step S205. Further, on setting the actual repetition frequency fr, the control unit 12 sets the tentative actual repetition frequency fr', which is obtained by multiplying the coefficient parameter $\alpha$ set by the first frequency sweeping after the occurrence of the aliasing ceases to be detected and the reference repetition frequency fi, as the actual repetition frequency fr in place of step S206.

The control unit 12, as described above, uniquely sets the velocity range of interest $\pm Vi$ based on the velocity-range-of-interest designating information supplied from the input unit 2, and at the same time, the control unit 12 uniquely obtains the reference repetition frequency fi with respect to the set velocity range of interest $\pm Vi$. Further, the control unit 12 gradually changes the tentative actual repetition frequency fr' through the frequency sweeping described above to detect the coefficient parameter $\alpha$ in the last frequency sweeping in which no occurrence of aliasing is detected, in other words, to detect the coefficient parameter $\alpha$ in the first frequency sweeping after the occurrence of aliasing ceases to be detected, and obtains the actual repetition frequency fr by multiplying the reference repetition frequency fi with the coefficient parameter $\alpha$. Therefore, the control unit 12 can set the detectable velocity range $\pm Vr$ to an appropriately wide range in comparison with the velocity range of interest $\pm Vi$ without changing the velocity range of interest $\pm Vi$ by calculating the maximum velocity Vr based on the actual repetition frequency fr. Here, the detectable velocity range $\pm Vr$ is not excessively wide in comparison with the velocity range of interest $\pm Vi$, though the detectable velocity range $\pm Vr$ has a sufficiently wide velocity range such that the velocity which is estimated to be within the velocity range of interest $\pm Vi$ does not change over the detectable velocity range. Therefore, the occurrence of the aliasing can be prevented during the display/output of the color Doppler image or the tissue Doppler image that indicates the velocity of the moving body without inconvenience in display of images such as the color Doppler image, the tissue Doppler image, and the color scale.

The control unit 12 desirably sets the coefficient parameter $\alpha$ to a real number within the range of two to four, so as to set the detectable velocity range to a suitable range. Here, the control unit 12 desirably changes the coefficient parameter $\alpha$ within the range of approximately 1 to 5 during the frequency sweeping mentioned above.

Further, the cutoff frequency fc is represented with the reference repetition frequency fi and the coefficient parameters $\alpha$ and $\beta$ as represented by expression (13). The control unit 12 can set an original velocity range of noises to be removed from the velocity range of interest $\pm Vi$ as the velocity range of noises to be removed from the detectable velocity range $\pm Vi$ by setting the cutoff frequency fc in the filter 72. Thus, the control unit 12 can set the detectable velocity range $\pm Vr$ having an equal or wider range than the velocity range of interest $\pm Vi$ without compromising the detecting capability with respect to velocities within a low velocity range, which is originally intended as the target of detection, within the velocity range of interest $\pm Vi$.

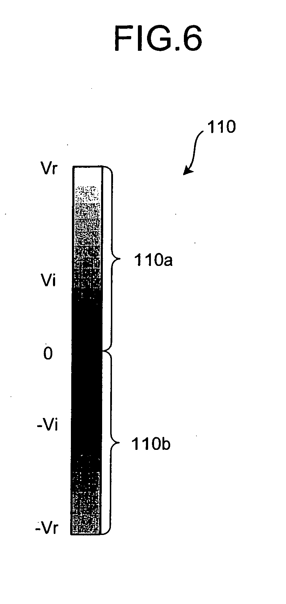

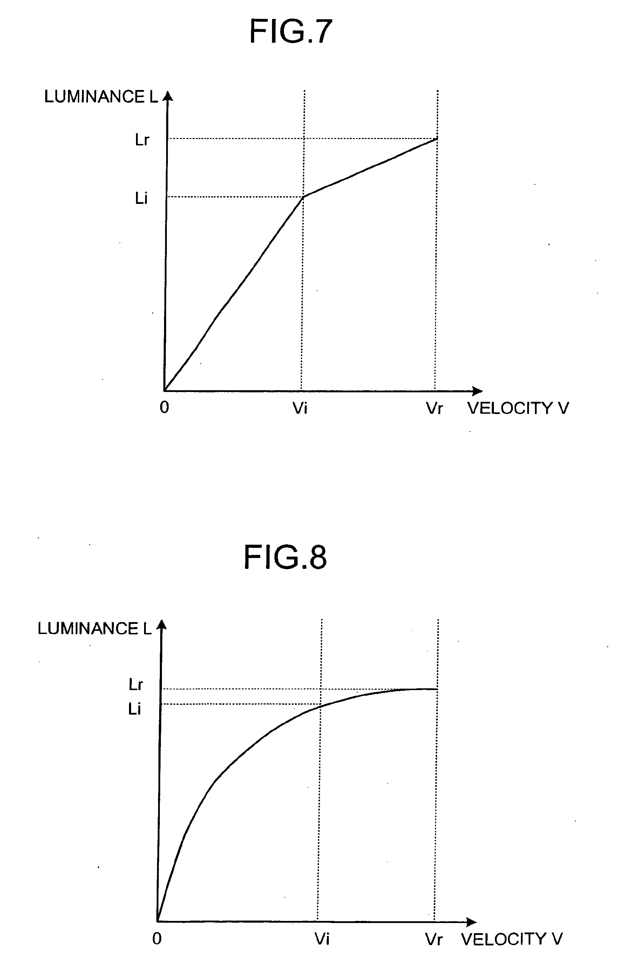

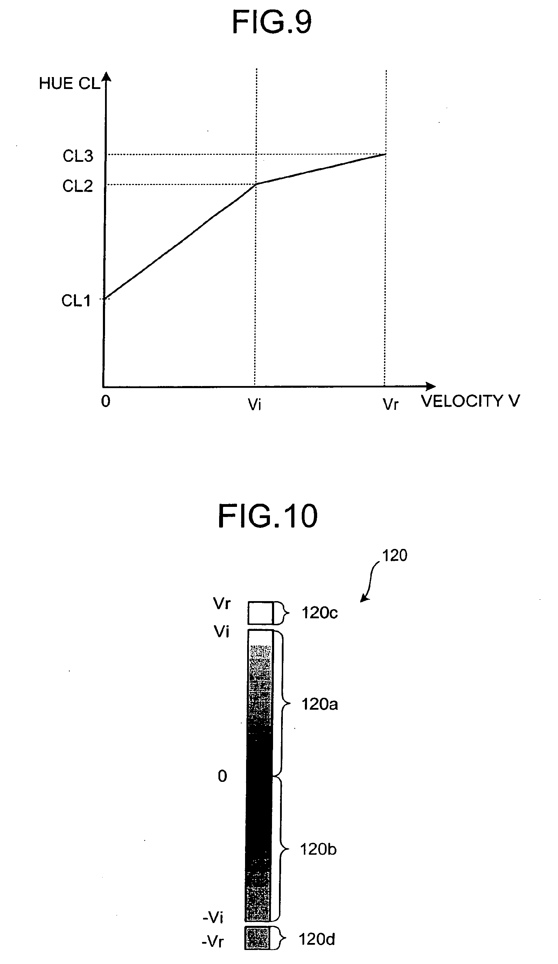

Processing performed by the color image data generator 8 to associate the color scale data mentioned above with the velocities will be described in detail. FIG. 6 is a schematic diagram illustrating an example of the color scale data associated with each velocity within the detectable velocity range $\pm Vr$. FIG. 7 is a schematic diagram illustrating an example of luminance variation in the color scale data corresponding to the variation in velocity. FIG. 8 is a schematic diagram illustrating another example of luminance variation in the color scale data corresponding to the variation in velocity. FIG. 9 is a schematic diagram illustrating an example of hue variation in the color scale data corresponding to the variation in velocity.

The color image data generator 8 allocates each level of luminance or hue in the color scale data to the velocity within the detectable velocity range $\pm Vr$, i.e., the velocity within the velocity range of interest $\pm Vi$, and to the velocity out of the velocity range of interest $\pm Vi$ though within the detectable velocity range $\pm Vr$, using the stored color scale data and the velocity range of interest $\pm Vi$ and the detectable velocity range $+Vr$ based on the respective parameter signals from the control unit 12 as described above. Thus, the color image data generator 8 generates color scale data 110 as shown in FIG. 6, for example.

The color scale data 110 consists of a color scale element 110a which corresponds to a positive velocity of the moving body, i.e., a positive velocity within the detectable velocity range $\pm Vr$, and a color scale element 110b which corresponds to a negative velocity of the moving body, i.e., a negative velocity within the detectable velocity range $\pm Vr$, as shown in FIG. 6. Here, the color scale element 110a corresponds to a positive velocity within the velocity range of interest $\pm Vi$, i.e., within the velocity range of 0 to Vi, and a positive velocity out of the velocity range of interest $\pm Vi$ though within the detectable velocity range $\pm Vr$, i.e., within the velocity range of Vi to Vr. The color scale element 110b corresponds to a negative velocity within the velocity range of interest $\pm Vi$, i.e., within the velocity range of 0 to -Vi, and a negative velocity out of the velocity range of interest $\pm Vi$ though within the detectable velocity range $\pm Vr$, i.e., within the velocity range of -Vr to -Vi.

For example, in the color scale element 110a, black color is allocated to the neighborhood of the zero velocity, i.e., to the velocity range of noises to be removed, and a gradation of colors ranging from red to yellow is allocated to the positive velocity range within the detectable velocity range $\pm Vr$. Further, in the color scale element 110b, black color is allocated to the neighborhood of the zero velocity, i.e., to the velocity range of noises to be removed, and a gradation of colors ranging from dark violet to light blue is allocated to the negative velocity range within the detectable velocity range $\pm Vr$.

Here, on allocating each level of luminance or hue within the color scale element 110a to the positive velocity within the detectable velocity range $\pm Vr$, the color image data generator 8 sets a wider luminance variation or hue variation for the positive velocity within the velocity range of interest $\pm Vi$ in comparison with the luminance variation or hue variation for the positive velocity out of the velocity range of interest ±Vi though within the detectable velocity range ±Vr.

For example, the color image data generator 8, as shown in FIG. 7, monotonously increases the luminance L of the color scale element 110a from zero to Li in a linear manner against the velocity range from zero to Vi, while monotonously increases the luminance L from Li to Lr against the velocity range ranging from Vi to Vr. Here, the color image data generator 8 sets a wider luminance variation against the velocity variation ranging from zero to Vi in comparison with the luminance variation against the velocity variation from Vi to Vr using the velocity Vi as a boundary value. Thus, the color image data generator 8 can narrow the luminance variation corresponding to the variation in the positive velocities out of the velocity range of interest ±Vi though within the detectable velocity range ±Vr, while widening the luminance variation corresponding to the variation in the positive velocities within the velocity range of interest ±Vi.

Further, the color image data generator 8, may monotonously increase the luminance L of the color scale element 110a from zero to Li as represented by a curve of FIG. 8 against the velocity range from zero to Vi, while monotonously increasing the luminance L from Li to Lr as represented by a curve of FIG. 8 against the velocity range from Vi to Vr. Further, the color image data generator 8 may set a wider luminance variation corresponding to the variation in velocities ranging from zero to Vi in comparison with the luminance variation corresponding to the variation in velocities ranging from Vi to Vr using the velocity Vi as a boundary value. Here, the color image data generator 8 can narrow the luminance variation corresponding to the variation in the positive velocities out of the velocity range of interest ±Vi though within the detectable velocity range ±Vr, while widening the luminance variation corresponding to the variation in the positive velocities within the velocity range of interest ±Vi.

Further, the color image data generator 8, as shown in FIG. 9, may monotonously change the level of the hue CL of the color scale element 110a from CL1 to CL2 in a linear manner against the velocity range from zero to Vi, while monotonously changes the level of the hue CL from CL2 to CL3 in a linear manner against the velocity range from Vi to Vr. Further, the color image data generator 8 may set a wider hue variation corresponding to the velocity changes within the velocity range from zero to Vi in comparison with the hue variation corresponding to the velocity changes within the velocity range from Vi to Vr using the velocity Vi as a boundary value. Here, the color image data generator 8 can narrow the hue variation corresponding to the variation in the positive velocities out of the velocity range of interest ±Vi though within the detectable velocity range ±Vr, while widening the hue variation corresponding to the variation in the positive velocities within the velocity range of interest ±Vi.

The color scale element 110b is data obtained by inverting the signs of the velocities associated with the color scale element 110a. Therefore, the color image data generator 8 can narrow the luminance variation or the hue variation corresponding to the variation in the negative velocities out of the velocity range of interest ±Vi though within the detectable velocity range ±Vr, while widening the luminance variation or the hue variation corresponding to the variation in the negative velocities within the velocity range of interest ±Vi in substantially the same manner as in the color scale element 110a.

The color image data generator 8 can associate a relatively moderate luminance variation or hue variation with the variation in velocities out of the velocity range of interest ±Vi and within the detectable velocity range ±Vr, while associating a relatively large luminance variation or hue variation with the variation in velocities within the velocity range of interest ±Vi by using the color scale data 110 consisting of the color scale element 110a and the color scale element 110b. Thus, the color image data generator 8 can generate color image data corresponding to the velocity image which allows the operator to easily recognize the velocity of interest of the moving body when the image is displayed/output on/to the monitor 10.

In the first embodiment of the present invention, the scale of the velocity variation is set constant over the entire velocity range of the detectable.velocity range ±Vr, i.e., over both the velocity range within the velocity range of interest ±Vi and the velocity range out of the velocity range of interest ±Vi though within the detectable velocity range ±Vr. The present invention, however, is not limited to the above. The scale of the velocity variation in the velocity range within the velocity range of interest± Vi may be set larger than the scale of the velocity variation in the velocity range out of the velocity range of interest ±Vi and within the detectable velocity range ±Vr.

FIG. 10 is a schematic diagram illustrating an example of the color scale data in which the scale of the variation in velocities within the velocity range of interest ±Vi is increased. Color scale data 120, as shown in FIG. 10, consists of a color scale element 120a corresponding to positive velocities within the velocity range of interest ±Vi and a color scale element 120b corresponding to negative velocities within the velocity range of interest ±Vi, a color scale element 120c corresponding to positive velocities out of the velocity range of interest ±Vi and within the detectable velocity range ±Vr, a color scale element 120d corresponding to negative velocities out of the velocity range of interest ±Vi and within the detectable velocity range ±Vr.

On allocating each level of luminance or hue in the color scale data 120 to the velocity within the detectable velocity range ±Vr, the color image data generator 8 reduces the scale of the velocity variation for the color scale elements 120c and 120d, while increasing the scale of the velocity variation for the color scale elements 120a and 120b. Thus, the color image data generator 8, as shown in FIG. 10, can generate the color scale data 120 in which the portion corresponding to the velocities within the velocity range of interest ±Vi is sufficiently wider than the portion corresponding to the velocities out of the velocity range of interest ±Vi and within the detectable velocity range ±Vr. In other words, the color image data generator 8 can surely generate the color scale data in which the portion corresponding to the velocities within the velocity range of interest ±Vi occupies a larger area than the other portions even when the detectable velocity range ±Vr is set even wider than the velocity range of interest ±Vi.

Then, the monitor 10 can display/output an image of the color scale indicating the color scale data so that the portion corresponding to the velocities within the velocity range of interest ±Vi occupies a larger area than other portions as illustrated by the color scale data 120. The operator can recognize the velocity out of the velocity range of interest ±Vi though within the detectable velocity range ±Vr and at the same time can securely and easily recognize the velocity within the velocity range of interest ±Vi, by referring to the color scale displayed/output.

The color image data generator 8 may divide the color scale element 120a and the color scale element 120c by setting the maximum velocity Vi as a boundary. Similarly, the color image data generator 8 may divide the color scale element 120b and the color scale element 120d by setting the minimum velocity -Vi as a boundary.

In the first embodiment of the present invention, the ultrasonic transducer 3 is realized with the array transducer. The present invention, however, is not limited thereto. The ultrasonic transducer 3 may include a rotary driving system and be driven mechanically to perform the ultrasonographic scanning.

Further, in the first embodiment of the present invention, the various types of information such as operation parameters are stored in each element. The present invention, however, is not limited thereto. Alternatively, the control unit 12 may collectively store and manage the various types of information.

Further, in the first embodiment of the present invention, the control unit 12 calculates the cutoff frequency fc and transmits the parameter signals indicating the cutoff frequency fc to the filter 72. The present invention, however, is not limited thereto. Alternatively, the control unit 12 may transmit the parameter signals each indicating the reference repetition frequency fi and the maximum velocities Vi, Vr to the filter 72, and the filter 72 may calculate the cutoff frequency fc based on the parameter signals sent from the control unit 12.

Further, in the first embodiment of the present invention, the control unit 12 gives a command to the filter 72 to set the cutoff frequency fc, and thereafter gives a command to the color image data generator 8 to associate the color scale data with the velocity. The present invention, however, is not limited thereto. Alternatively, the control unit 12 may give a command to the color image data generator 8 to associate the color scale data with the velocity, and thereafter, or simultaneously, give a command to the filter 72 to set the cutoff frequency fc.

Further, in the first embodiment of the present invention, the luminance or the hue of the color scale data is varied corresponding to the variation in velocities that are out of the velocity range of interest ±Vi and within the detectable velocity range ±Vr. The present invention, however, is not limited thereto. Alternatively, a constant level of the luminance or the hue of the color scale data may be allocated to the velocity variation out of the velocity range of interest ±Vi and within the detectable velocity range ±Vr.

Further, in the first embodiment of the present invention, the hue is changed linearly corresponding to the variation in velocities within the detectable velocity range ±Vr. The present invention, however, is not limited thereto. Alternatively, the hue may be changed in a curved manner corresponding to the variation in velocities within the detectable velocity range ±Vr, if the hue variation corresponding to the variation in velocities within the velocity range of interest ±Vi is large in comparison with the hue variation corresponding to the variation in velocities out of the velocity range of interest ±Vi and within the detectable velocity range ±Vr.

As described above, in the first embodiment of the present invention, the velocity range of interest is uniquely set based on the velocity-range-of-interest designating information supplied by the operator through the input manipulation, and the variable detectable velocity range is set as a wider velocity range than the velocity range of interest so as to cover the velocity range of interest. Further, the velocity within the detectable velocity range and out of the velocity range that corresponds to the velocity range of interest and that is in the neighborhood of the zero velocity is calculated as the velocity of the desired moving body that moves within the subject body. Therefore, the first embodiment can realize an ultrasonic diagnostic apparatus which can suppress the occurrence of aliasing at the detection of the velocity of the desired moving body without compromising the capability to detect a velocity within a desired low velocity range within the velocity range of interest.

Further, a desired level of luminance or hue in the color scale data is allocated to a velocity within the detectable velocity range, and the velocity image indicating the velocity of the desired moving body is generated and output based on the allocated color scale data and the velocity calculated as the velocity of the desired moving body. Therefore, the first embodiment can realize an ultrasonic diagnostic apparatus which can surely display the velocity image indicating the velocity within the detectable velocity range on the monitor without causing the occurrence of aliasing.

Further, the luminance variation or the hue variation corresponding to the velocity variation is set larger for the velocity within the velocity range of interest than for the velocity out of the velocity range of interest and within the detectable velocity range. Therefore, it is possible to display the velocity image, which allows for the operator to easily recognize the velocity within the velocity range of interest, on the screen.

Further, the scale of the variation in velocities out of the velocity range of interest and within the detectable velocity range can be reduced while the scale of the variation in velocities within the velocity range of interest is increased. Then, the width of the color scale data corresponding to the velocities within the velocity range of interest can be made sufficiently longer than the width of the color scale data corresponding to the velocities out of the velocity range of interest and within the detectable velocity range. Thus, the image displayed on the screen can be made to have a color scale in which a portion occupied by the color scale data corresponding to the velocities within the velocity range of interest is sufficiently larger than other portions. Therefore, the operator can recognize the velocities out of the velocity range of interest and within the detectable velocity range and can securely and easily recognize the velocity within the velocity range of interest by referring to the image of such a color scale.

Second Embodiment

A second embodiment of the present invention will be described in detail below. In the first embodiment described above, a certain level of luminance or hue in the color scale data is allocated to each velocity within the detectable velocity range ±Vr, and the displayed/output image has the color scale corresponding to all the velocities within the detectable velocity range ±Vr. In the second embodiment, however, luminance or hue in the color scale data is not allocated to the velocity out of the velocity range of interest ±Vi and within the detectable velocity range ±Vr, while the luminance or the hue in the color scale data is allocated to each velocity within the velocity range of interest ±Vi, and the displayed/output image has a color scale corresponding to all velocities within the velocity range of interest ±Vi.

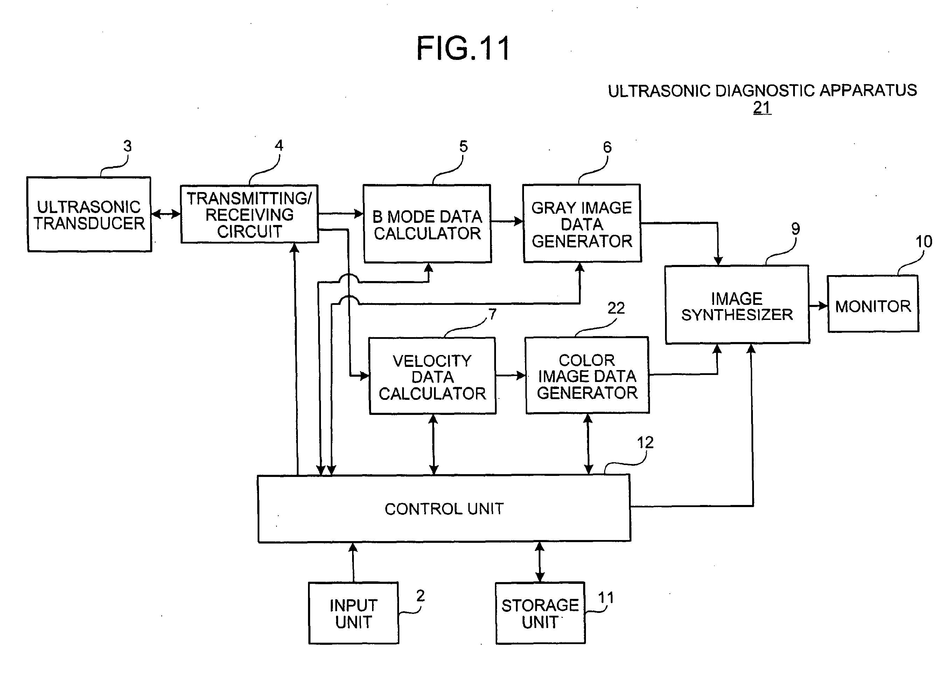

FIG. 11 is a block diagram illustrating an exemplary structure of an ultrasonic diagnostic apparatus according to the second embodiment of the present invention. An ultrasonic diagnostic apparatus 21 includes a color image data generator 22 in place of the color image data generator 8. In other respects, the structure of the ultrasonic diagnostic apparatus of the second embodiment is the same as the structure of the ultrasonic diagnostic apparatus of the first embodiment, and the same element is denoted by the same reference character.

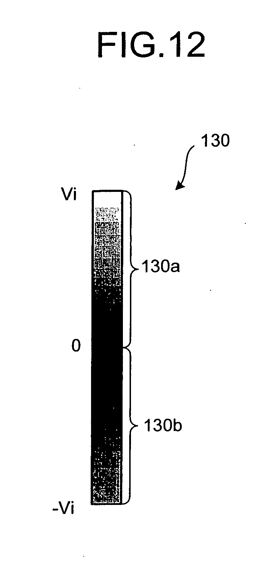

FIG. 12 is a schematic diagram illustrating an example of color scale data associated with velocities within the velocity range of interest ±Vi. The color image data generator 22 has substantially the same function and structure as those of the color image data generator 8 described above. Further, on receiving the parameter signals indicating the velocity range of interest ±Vi and the detectable velocity range ±Vr from the control unit 12, the color image data generator 22 allocates a certain level of luminance or hue in the color scale data to each velocity within the velocity range of interest ±Vi based on the stored color scale data and the parameter signals under the control of the control unit 12. Thus, the color image data generator 22 generates color scale data 130 shown in FIG. 12, for example.

The color scale data 130, as shown in FIG. 12, consists of a color scale element 130a corresponding to positive velocities within the velocity range of interest ±Vi, i.e., the velocity range from 0 to Vi, and a color scale element 110b corresponding to negative velocities within the velocity range of interest ±Vir, i.e., the velocity range from 0 to -Vi. For example, in the color scale element 130a, a black color is allocated to a velocity range in the neighborhood of the zero velocity, i.e., a range of velocities to be removed as noises, and a gradation of colors ranging from red to yellow is allocated to the positive velocities within the velocity range of interest ±Vi. Further, in the color scale element 130b, a black color is allocated to a velocity range in the neighborhood of the zero velocity, i.e., a range of velocities to be removed as noises, while a gradation of colors ranging from dark violet to light blue is allocated to the negative velocities within the velocity range of interest ±Vi. Here, the color image data generator 22 allocates substantially all the levels of luminance or substantially all the levels of hue in the stored color scale data to the velocity range of interest ±Vi.

Further, on receiving the velocity data of interest from the velocity data calculator 7, the color image data generator 22 classifies the velocity of the moving body based on the velocity data of interest into either the velocity within the velocity range of interest ±Vi or the velocity out of the velocity range of interest ±Vi and within the detectable velocity range ±Vr. Further, on obtaining the velocity of the moving body based on the velocity data of interest as the velocity within the velocity range of interest ±Vi, the color image data generator 22 obtains color image data corresponding to the obtained velocity using the color scale data associated with each velocity within the velocity range of interest ±Vi and the velocity data of interest. Then, the color image data generator 22 transmits the obtained color image data to the image synthesizer 9. Then, the monitor 10 can display/output a velocity image indicating the obtained velocity as a color Doppler image or a tissue Doppler image. Further, when the color image data generator 22 transmits image data corresponding to the color scale data associated with the velocities within the velocity range of interest ±Vi as exemplified by the color scale data 130 to the image synthesizer 9, the monitor 10 can display/output an image of a color scale indicating the color scale data on the same screen on which the color Doppler image or the tissue Doppler image is shown to indicate the obtained velocity.

On the other hand, the color image data generator 22 allocates a predetermined color, such as a black color to velocities out of the velocity range of interest ±Vi and within the detectable velocity range,+Vr. Therefore, when the obtained velocity of the moving body based on the velocity data of interest sent from the velocity data calculator 7 is the velocity out of the velocity range of interest ±Vi and within the detectable velocity range ±Vr, the color image data generator 22 converts the velocity data of interest and obtains image data in which the black color is allocated to the obtained velocity. Here, the color image data generator 22 classifies the velocity of the moving body into either the velocity within the velocity range of interest ±Vi or the velocity out of the velocity range of interest ±Vi and within the detectable velocity range ±Vr, and at the same time, the color image data generator 22 allocates the black color to the velocity based on the velocity data of interest without using the color scale data associated with the velocities within the velocity range of interest ±Vi. Therefore, the color image data generator 22 does not cause the aliasing mentioned above, even when the velocity obtained from the velocity data calculator 7 is out of the velocity range of interest ±Vi and within the detectable velocity range ±Vr.

Further, the color image data generator 22 transmits the obtained image data to the image synthesizer 9 as the non-target color image data. The image synthesizer 9 overwrites the non-target color image data with gray image data described above to obtain synthesized image data. Here, the monitor 10 does not display the color Doppler image or the tissue Doppler image that indicates the velocity of the moving body, whose velocity is out of the velocity range of interest ±Vi and within the detectable velocity range ±Vr.

As described above, the second embodiment of the present invention has substantially the same function and structure as those of the first embodiment described above. Further, all the levels of luminance or hue in the color scale data are allocated to the velocities within the set velocity range of interest, and a predetermined color, such as a black color is allocated to the velocities out of the velocity range of interest and within the detectable velocity range. Still further, the calculated velocity of the moving body is classified into either the velocity within the velocity range of interest or the velocity out of the velocity range of interest and within the detectable velocity range. When the velocity of the moving body is classified into the velocity within the velocity range of interest, the velocity image is generated and output. On the other hand, when the velocity of the moving body is classified into the velocity out of the velocity range of interest and within the detectable velocity range, the velocity image is not displayed nor output. Therefore, the occurrence of aliasing can be suppressed with respect to the calculated velocity of the moving body, while the velocity image is not displayed on the screen for the velocity of the moving body out of the velocity range of interest and within the detectable velocity range, and the velocity image can be displayed on the screen for the velocity of the moving body within the velocity range of interest. Thus, the second embodiment can enjoy substantially the same advantages as those of the first embodiment. In addition, the second embodiment can realize an ultrasonic diagnostic apparatus which allows for a readily recognition of the velocity of a desired moving body whose velocity is within the velocity range of interest.

INDUSTRIAL APPLICABILITY

As can be seen from the foregoing, the ultrasonic diagnostic apparatus according to the present invention is useful for examining an interior of a living body through observation of a velocity and a direction of motion of a moving body inside the living body, and in particular, suitable for an ultrasonic diagnostic apparatus for observing a velocity and a direction of motion of a moving body, such as blood and organs, whose velocity has a wide range to be detected.

**Claims**

1. An ultrasonic diagnostic apparatus transmitting/receiving ultrasound to an interior of a subject body plural times to obtain plural pieces of ultrasound data, generating and outputting an ultrasound tomographic image of the interior of the subject body based on the obtained ultrasound data, calculating a velocity of a moving body that moves in the subject body as a velocity within a predetermined velocity range, and generating and outputting a velocity image that indicates the velocity of the moving body based on the calculated velocity and color scale data, the ultrasonic diagnostic apparatus comprising:

   an input unit that supplies information indicating an velocity range of interest of the moving body as an input;
   a velocity range setting control unit that sets a variable detectable velocity range as the predetermined velocity range based on the information supplied from the input unit, the variable detectable velocity range being a wider velocity range than the velocity range of interest and covering the velocity range of interest; and
   an image processing control unit that allocates the color scale data to each velocity within the detectable velocity range to generate the velocity image based on the color scale data allocated and the calculated velocity.

2. The ultrasonic diagnostic apparatus according to claim 1, wherein
the velocity range setting control unit sets the detectable velocity range, in which a velocity range that is in a neighborhood of zero and that corresponds to the velocity range of interest is set for removal, as the predetermined velocity range.

3. An ultrasonic diagnostic apparatus transmitting/receiving ultrasound to an interior of a subject body plural times to obtain plural pieces of ultrasound data, generating and outputting an ultrasound tomographic image of the interior of the subject body based on the obtained ultrasound data, calculating a velocity of a moving body that moves in the subject body as a velocity within a predetermined velocity range, and generating and outputting a velocity image that indicates the velocity of the moving body based on the calculated velocity and color scale data, the ultrasonic diagnostic apparatus comprising:

an input unit that supplies information indicating an velocity range of interest of the moving body as an input; and a velocity range setting control unit that sets a variable detectable velocity range as the predetermined velocity range based on the information supplied from the input unit, the variable detectable velocity range being a wider velocity range than the velocity range of interest and covering the velocity range of interest, and the detectable velocity range including a velocity range, which is in a neighborhood of zero and corresponds to the velocity range of interest, for removal.

4. The ultrasonic diagnostic apparatus according to claim 1 or 2, further comprising a display unit that displays and outputs plural types of images simultaneously, the images including a color scale image corresponding to the color scale data allocated to each velocity within the detectable velocity range and the velocity image, wherein the image processing control unit controls the display unit to display the plural types of images.

5. The ultrasonic diagnostic apparatus according to claim 4, wherein the image processing control unit controls the display unit to reduce the color scale image corresponding to a velocity out of the velocity range of interest and within the detectable velocity range to a smaller size than a size of the color scale image corresponding to a velocity within the velocity range of interest.

6. The ultrasonic diagnostic apparatus according to any one of claims 1 to 3, wherein the image processing control unit, on allocating the color scale data to each velocity within the detectable velocity range, sets hue variation or luminance variation of the color scale data corresponding to variation in velocities within the velocity range of interest larger than the hue variation or the luminance variation of the color scale data corresponding to variation in velocities out of the velocity range of interest and within the detectable velocity range.

7. The ultrasonic diagnostic apparatus according to any one of claims 1 to 3, wherein the velocity range setting control unit includes a transmission/reception control unit which sets the velocity range of interest based on the information supplied from the input unit, calculates a reference repetition frequency corresponding to a maximum velocity value within the velocity range of interest and a tentative repetition frequency related with a number of repetitions of transmission and reception of the ultrasound, performs frequency sweeping to sweep the tentative repetition frequency, and sequentially controls the transmission and the reception of the ultrasound using the tentative repetition frequency for each frequency sweeping, and a velocity calculation control unit which sequentially calculates the velocity of the moving body based on the plural pieces of ultrasound data obtained through the control of the transmission and the reception of the ultrasound by the transmission/reception control unit, wherein the transmission/reception control unit sequentially detects the velocities of the moving body from the velocity calculation control unit, performs an aliasing determination to sequentially determine whether the aliasing occurs or not based on the sequentially detected velocities of the moving body, and sets an actual repetition frequency and the detectable velocity range for the control of the transmission and the reception of the ultrasound based on a result of the aliasing determination, and the velocity calculation control unit sets a velocity range to remove a velocity range portion that corresponds to the velocity range of interest and is in a neighborhood of zero from the detectable velocity range, using at least the reference repetition frequency.

# FIG.1

ULTRASONIC DIAGNOSTIC APPARATUS
1

# FIG.2

VELOCITY DATA CALCULATOR 7

FROM TRANSMITTING/ RECEIVING CIRCUIT 4 → COMPLEX SIGNAL GENERATING CIRCUIT 71 → FILTER 72 → AUTOCOR-RELATION CIRCUIT 73 → MOTION INFORMATION CALCULATOR 74 → THRESHOLD PROCESSING CIRCUIT 75 → TO COLOR IMAGE DATA GENERATOR 8

CONNECTED TO CONTROL UNIT 12

EP 1 769 746 A1

# FIG.3

START

S101
IS VELOCITY-RANGE-OF-INTEREST DESIGNATING INFORMATION DETECTED? — NO

YES

CALCULATE REFERENCE REPETITION FREQUENCY — S102

ACTUAL REPETITION FREQUENCY SETTING PROCESS — S103

SET DETECTABLE VELOCITY RANGE — S104

GIVE COMMAND TO SET CUTOFF FREQUENCY — S105

GIVE COMMAND ON ASSOCIATION OF COLOR SCALE DATA WITH VELOCITIES — S106

S107
IS COMPLETION OF SETTING DETECTED? — NO

YES

GIVE COMMAND TO TRANSMIT/RECEIVE ELECTRIC PULSE SIGNALS — S108

GIVE COMMAND TO DISPLAY IMAGE — S109

S110
IS COMMAND INFORMATION DETECTED? — NO

YES

VELOCITY RANGE OF INTEREST DESIGNATING INFORMATION

S111
WHAT IS DETECTED COMMAND INFORMATION?

ENDING COMMAND INFORMATION

END

# FIG.4

# FIG.5

```
┌─────────────────────────────────────────────┐
│  ACTUAL REPETITION FREQUENCY SETTING PROCESS  │
└─────────────────────────────────────────────┘
                      │
                      ▼
┌─────────────────────────────────────────────┐  S201
│   CALCULATE TENTATIVE ACTUAL REPETITION       │
│                FREQUENCY                       │
└─────────────────────────────────────────────┘
                      │
                      ▼
┌─────────────────────────────────────────────┐  S202
│           FREQUENCY SWEEPING                  │
└─────────────────────────────────────────────┘
                      │
                      ▼
┌─────────────────────────────────────────────┐  S203
│   SET TENTATIVE DETECTABLE VELOCITY RANGE     │
└─────────────────────────────────────────────┘
                      │
                      ▼
┌─────────────────────────────────────────────┐  S204
│             DETECT VELOCITY                    │
└─────────────────────────────────────────────┘
                      │
                      ▼
                    IS                           S205
             OCCURRENCE OF ALIASING                    NO
                 DETECTED?
                      │ YES
                      ▼
┌─────────────────────────────────────────────┐  S206
│      SET ACTUAL REPETITION FREQUENCY          │
└─────────────────────────────────────────────┘
                      │
                      ▼
              ┌──────────────┐
              │    RETURN     │
              └──────────────┘
```

# FIG.6

# FIG.7

# FIG.8

# FIG.9

HUE CL

CL3

CL2

CL1

0    Vi    Vr    VELOCITY V

# FIG.10

120

Vr    □ } 120c

Vi

120a

0

120b

-Vi

-Vr   ▨ } 120d

# FIG.11

EP 1 769 746 A1

ULTRASONIC DIAGNOSTIC APPARATUS
21

# FIG.12

130

Vi

130a

0

130b

-Vi

| | | International application No. |
|---|---|---|
| | **INTERNATIONAL SEARCH REPORT** | PCT/JP2005/011763 |

| A. CLASSIFICATION OF SUBJECT MATTER |
|---|
| Int.Cl$^7$ A61B8/06 |
| |
| According to International Patent Classification (IPC) or to both national classification and IPC |

| B. FIELDS SEARCHED |
|---|
| Minimum documentation searched (classification system followed by classification symbols) |
| Int.Cl$^7$ A61B8/00-8/15 |
| |
| Documentation searched other than minimum documentation to the extent that such documents are included in the fields searched |
| Jitsuyo Shinan Koho        1922-1996   Jitsuyo Shinan Toroku Koho    1996-2005 |
| Kokai Jitsuyo Shinan Koho   1971-2005   Toroku Jitsuyo Shinan Koho    1994-2005 |
| Electronic data base consulted during the international search (name of data base and, where practicable, search terms used) |
| |

| C. DOCUMENTS CONSIDERED TO BE RELEVANT | | |
|---|---|---|
| Category* | Citation of document, with indication, where appropriate, of the relevant passages | Relevant to claim No. |
| X<br>Y<br>A | JP 10-314170 A  (Toshiba Corp.),<br>02 December, 1998 (02.12.98),<br>Full text; all drawings<br>(Family: none) | 1,4,5,6<br>2,3<br>7 |
| Y<br>A | JP 6-245933 A  (Toshiba Corp.),<br>06 September, 1994 (06.09.94),<br>Full text; all drawings<br>& US 5441052 A | 2,3<br>1,4-7 |
| A | JP 6-186 A  (Toshiba Corp.),<br>11 January, 1994 (11.01.94),<br>Full text; all drawings<br>(Family: none) | 1-7 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 30 August, 2005 (30.08.05) | 20 September, 2005 (20.09.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

| International application No. |
| --- |
| PCT/JP2005/011763 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2004-154475 A  (Toshiba Corp.),<br>03 June, 2004 (03.06.04),<br>Full text; all drawings<br>(Family: none) | 1-7 |
| A | JP 63-317142 A  (Toshiba Corp.),<br>26 December, 1988 (26.12.88),<br>Full text; all drawings<br>(Family: none) | 1-7 |

Form PCT/ISA/210 (continuation of second sheet) (January 2004)

**REFERENCES CITED IN THE DESCRIPTION**

**Patent documents cited in the description**

- JP H11146879 A

- JP H06002134 B